(19)

Europäisches Patentamt
European Patent Office
Office européen des brevets

(11) **EP 4 069 423 B1**

(12) **EUROPEAN PATENT SPECIFICATION**

(45) Date of publication and mention
of the grant of the patent:
**04.03.2026 Bulletin 2026/10**

(21) Application number: **20817349.2**

(22) Date of filing: **04.12.2020**

(51) International Patent Classification (IPC):
*B01L 3/00* (2006.01)    *C12M 3/06* (2006.01)

(52) Cooperative Patent Classification (CPC):
**B01L 3/502761; C12M 23/16;** B01L 2200/0652;
B01L 2200/0684; B01L 2300/0864; B01L 2300/087;
B01L 2300/161; B01L 2300/163; B01L 2400/0655

(86) International application number:
**PCT/EP2020/084589**

(87) International publication number:
**WO 2021/110896 (10.06.2021 Gazette 2021/23)**

(54) **MICROFLUIDIC DEVICE AND METHOD FOR PROCESSING PARTICLES**

MIKROFLUIDISCHE VORRICHTUNG UND VERFAHREN ZUR VERARBEITUNG VON PARTIKELN

DISPOSITIF MICROFLUIDIQUE ET PROCÉDÉ DE TRAITEMENT DE PARTICULES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.12.2019 EP 19306568**

(43) Date of publication of application:
**12.10.2022 Bulletin 2022/41**

(73) Proprietor: **Astraveus**
**94270 Le Kremlin-Bicêtre (FR)**

(72) Inventors:
• **LAURENT, Jérémie**
**69100 Villeurbanne (FR)**
• **HOU, Xue**
**92150 Suresnes (FR)**

(74) Representative: **Icosa**
**83 avenue Denfert-Rochereau**
**75014 Paris (FR)**

(56) References cited:
EP-A2- 2 970 849    US-A1- 2010 035 292
US-A1- 2014 162 262    US-B1- 6 551 841

• GREGORY A. COOKSEY ET AL: "A multi-purpose
microfluidic perfusion system with
combinatorial choice of inputs, mixtures,
gradient patterns, and flow rates", LAB ON A
CHIP, vol. 9, no. 3, 21 April 2010 (2010-04-21),
pages 417 - 426, XP055534091, ISSN: 1473-0197,
DOI: 10.1039/B806803H

## Description

The present invention relates to a microfluidic device and a method using the same for processing particles, in particular cells. The invention more particularly relates to a microfluidic device and a method using the same for realizing various types of bioproduction operations on living cells, such as filtration, seeding, amplification, transduction, sorting, harvest, etc.

## BACKGROUND

In recent years, microfluidic "chip" technology has been widely applied for cell suspension processing. However, in existing cell suspension processing microfluidic devices, input suspension dispersion limits the efficacy and the accuracy of the procedures. Indeed, cells are complex systems that are sensitive to many parameters of their environment. As a result, small changes in the execution of a bioproduction protocol may have a big impact. Similarly, parameter heterogeneity within a bioproduction step, meaning that cells individually experience different values of important parameters, can result in a very heterogeneous output. Heterogeneity also implies that some cells experience parameters different from the optimal ones, which can result in reduced efficiency and efficacy. Heterogeneity also prevents from efficiently identifying optimal process parameters, since the measured output is representative of a spread distribution of parameters and thus provides limited amount of information regarding the relevance of the targeted set of parameters. As a result, inter-batch reproducibility and intra-batch homogeneity of parameters are key in bioproduction to obtain reproducible and homogeneous outputs. In turn, this makes it possible to obtain reliable data from protocol parameters screening so as to determine optimal process conditions providing maximal efficiency, efficacy and reproducibility.

In addition, few microfluidic bioproduction devices are configured to support bioproduction including "culture steps", such as amplification and differentiation steps. The existing microfluidic devices supporting such general bioprocessing steps do not offer cell and reactant efficiency for a reasonable complexity and cost, while providing a sufficient capacity. An example of a microfluidic device for processing cells is known from US2010/035292 A1.

It is these drawbacks that the invention is intended more particularly to remedy by proposing a microfluidic device and a method for processing particles, in particular cells, which enable cheap, efficacious, efficient in terms of quantity of particles and reactant, and accurate bioprocessing technologies.

## SUMMARY

For this purpose, according to one aspect, a subject of the invention is a microfluidic device for processing particles, in particular cells, comprising:

- a processing chamber including at least two elongated segments,
- at least one input seeding channel and one output seeding channel configured to define a seeding flow in a transverse direction to the longitudinal direction of all segments of the processing chamber, and
- at least one input harvest channel and one output harvest channel configured to define a harvest flow in the longitudinal direction of all segments of the processing chamber,

wherein the microfluidic device comprises:

- for a first segment S1 of the processing chamber, a plurality of input seeding channels whose junctions with S1 are distributed along S1, the plurality of input seeding channels being defined by a single input seeding tree;
- for a second segment S2 of the processing chamber, different from S1, a plurality of output seeding channels whose junctions with S2 are distributed along S2, the plurality of output seeding channels being defined by a single output seeding tree; and
- one connection channel or a plurality of connection channels, each connection channel having junctions with two distinct segments of the processing chamber, the connection channel or plurality of connection channels being configured to allow the seeding flow through all the segments serially.

Within the meaning of the invention, a processing chamber is a volume of the microfluidic device in which a bioprocess may be implemented. The chamber may be the sum of several segments in fluidic communication together, the segmentation of the chamber being useful for some flow steps such as seeding and harvesting. As segments are in fluidic communication, the chamber may be defined as the volume in which bioprocess parameters may be kept the same, regardless segmentation. In some specific cases, a step of bioprocess may be implemented in a single segment of a chamber.

Within the meaning of the invention, a flow is serial through several segments when the flow front is flowing successively through each segment: in other words, flow crosses the first segment, then reaches and crosses the second segment and so on until flow reaches the last segment. In particular, for seeding flow, serial connection between segments is allowed by connection elements; and for harvest flow, serial connection between segments is allowed by fluidic connectors. So as to allow the seeding flow to flow serially through all segments, each segment is:

- in fluidic connection with two or more distinct segments through connection channels; or
- in fluidic connection with one or more distinct segment through connection channels and in junction with a seeding tree (input or output).

Connection channels may be permanent or activated.

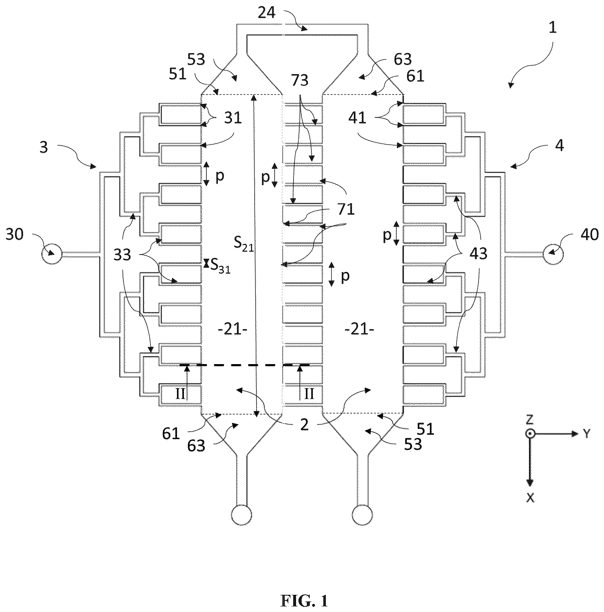

Permanent connection channels are defined by the geometry of the microfluidic device, with a specific length width and height (*i.e.* section), and junctions with two distinct segments. They are shown in Figures **1, 3, 4a** and **5.**

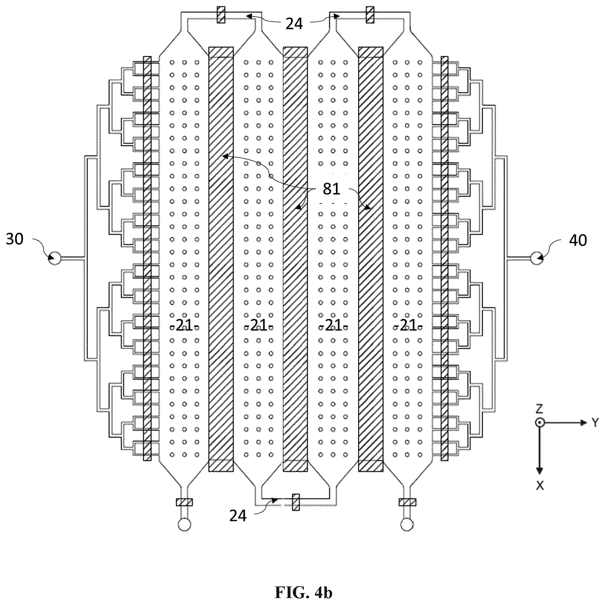

Activated connection channels are deformable parts of the chamber that may be pinched out, under application of an external pressure for instance. In their undeformed state, activated connection channels have a thickness comparable to the thickness of the chamber, but define limits between elongated segments. In their pinched state, activated connection channels define one thin and very large channel between two segments or a plurality of thin channels between two segments, the junctions between connection channels and segments being the areas where thickness changes from segment thickness to connection channel thickness. In a particular configuration, the chamber is split in two segments by a single activated connection channel defining a single large connection channel when pinched. In another configuration, the chamber is split in two segments by a single activated connection channel defining a plurality of narrow connection channels when pinched. In another configuration, the chamber is split in N segments (N greater than 2) by N-1 activated connection channels defining each a single large connection channel when pinched. Such a configuration (with N=4) is shown in Figure **4b.**

With the above features, the invention has the following advantages:

- the processing chamber, comprising at least two elongated segments, promotes a high differential of shear rate in the seeding channels and in the chamber during seeding, thus increasing the robustness of the seeding and promoting a high yield on the particles handled; within the meaning of the invention a segment is considered as elongated when its length is at least twice its width, preferably four times its width;
- the elongated segments being seeded serially, one single input seeding tree and one single output seeding tree with a simple geometry and a reduced footprint are required to seed the whole processing chamber, leading to an increase of the useful surface of the microfluidic device, *i.e.* the surface of processing chamber. Indeed, with such configuration, the surface of processing chamber may represent more than 45% of the whole surface of the microfluidic device, even more than 50%, 55%, 60% or 70%.

According to one feature, the at least two elongated segments of the processing chamber are parallel along their longitudinal direction.

According to one feature, the at least two elongated segments of the processing chamber have the same geometry.

According to one feature, the ratio *Rs_input* such that:

$$Rs\_input = \frac{S_{21}}{(\sum_k V_k * S_k)/V_{TOTs\_input}}$$

is higher than 50, preferably higher than 75, preferably higher than 100, preferably higher than 150, preferably higher than 200, where $S_{21}$ is the cross section of the segment perpendicular to the transverse direction, and $(\Sigma_k V_k * S_k)/V_{TOTs\_input}$ is the sum, for all input seeding channels between the first node closest to the tree root of the input seeding tree and the first segment of the processing chamber, of the products of the volume and the cross section of the input seeding channel, divided by the total volume $V_{TOTs\_input}$ which is the sum of the volumes of these input seeding channels.

According to one feature, the ratio *Rh_input* such that:

$$Rh\_input = \frac{W}{(\sum_j V_j * S_j)/V_{TOTh\_input}}$$

is lower than 20, preferably lower than 15, preferably lower than 10, preferably lower than 5, where W is the cross section of the segment perpendicular to the longitudinal direction, and $(\Sigma_j V_j * S_j)/V_{TOTh\_input}$ is the sum, for all input harvest channels between the first node closest to the tree root of the input harvest tree and the segment, of the products of the volume and the cross section of the input harvest channel, divided by the total volume $V_{TOTh\_input}$ which is the sum of the volumes of these input harvest channels.

With the above features, the invention has the following advantages:

- the ratio *Rs_input* for the seeding having a value higher than 50, preferably higher than 75, preferably higher than 100, preferably higher than 150, preferably higher than 200, induces a large variation in the shear rate between the seeding channels and the first segment of the processing chamber during a seeding flow, thus enhancing the seeding efficiency by promoting deposition of the particles in the processing chamber;
- the ratio *Rh_input* for the harvest having a value lower than 20, preferably lower than 15, preferably lower than 10, preferably lower than 5, induces a small variation in the shear rate between the harvest channels and the processing chamber during a harvest flow, thus enhancing the harvest efficiency.

According to one feature, the ratio *Rs_output* such that:

$$Rs\_output = \frac{S_{21}}{(\sum_k V_k * S_k)/V_{TOTs\_output}}$$

is higher than 50, preferably higher than 75, preferably higher than 100, preferably higher than 150, preferably higher than 200, where $S_{21}$ is the cross section of the segment perpendicular to the transverse direction, and $(\sum_k V_k * S_k)/V_{TOTs\_output}$ is the sum, for all output seeding channels between the first node closest to the tree root of the output seeding tree and the second segment of the processing chamber, of the products of the volume and the cross section of the output seeding channel, divided by the total volume $V_{TOTs\_output}$ which is the sum of the volumes of these output seeding channels.

According to one feature, the ratio *Rh_output* such that:

$$Rh\_output = \frac{W}{(\sum_j V_j * S_j)/V_{TOTh\_output}}$$

is lower than 20, preferably lower than 15, preferably lower than 10, preferably lower than 5, where W is the cross section of the segment perpendicular to the longitudinal direction, and $(\sum_j V_j * S_j)/V_{TOTh\_output}$ is the sum, for all output harvest channels between the first node closest to the tree root of the output harvest tree and the segment, of the products of the volume and the cross section of the output harvest channel, divided by the total volume $V_{TOTh\_output}$ which is the sum of the volumes of these output harvest channels.

According to one feature, the ratio *Rs_connect* such that:

$$Rs\_connect = \frac{S_{21}}{(\sum_k V_k * S_k)/V_{TOTs\_connect}}$$

is higher than 50, preferably higher than 75, preferably higher than 100, preferably higher than 150, preferably higher than 200, where S21 is the cross section of the segment perpendicular to the transverse direction, and $(\sum_k V_k * S_k)/V_{TOTs\_connect}$ is the sum, for all connection channels perfusing the segment of the processing chamber on one of its side, of the products of the volume and the cross section of the connection channels, divided by the total volume $V_{TOTs\_connect}$ which is the sum of the volumes of these connection channels.

Configurations in which the output seeding tree and the output harvest tree are such that each of the ratios *Rs_output* and *Rh_output* is of the same order of magnitude as the corresponding ratio *Rs_input* or *Rh_input,* enhances the symmetry of the microfluidic device and its efficiency for the seeding and harvest.

It is noted that the expression "harvest tree" as used herein can designate a unique harvest channel, in the case where there is only one harvest channel without real tree of harvest channels. In this case, the first node of the harvest tree is considered as being the geometrical transition between the segment and the harvest channel (as shown in Figure 1).

According to one embodiment, for the first segment S1 of the processing chamber, the input seeding tree has, on each possible flow path from the tree root to a junction with S1, at least one location such that the ratio of the seeding shear indicator SSI of the input seeding channel at this location to the average seeding shear indicator SSI of S1 is higher than 10, preferably higher than 20, more preferably higher than 40, where the seeding shear indicator of a channel or a segment is defined by:

$$SSI = \frac{T_Q}{S * h}$$

with $T_Q$ the percentage of the seeding flow flowing through the considered input seeding channel or segment, S the cross-section surface area of the input seeding channel or segment taken perpendicular to its longitudinal fiber for the seeding flow, and h the height of the input seeding channel or segment taken in the height direction.

According to an embodiment, for the connection channels establishing a fluidic connection between two segments of the processing chamber, the seeding shear indicator SSI of the connecting channel to the average seeding shear indicator SSI of each segment of the processing chamber is higher than 10, preferably higher than 20, more preferably higher than 40, where the seeding shear indicator of a connection channel or a segment is defined by:

$$SSI = \frac{T_Q}{S * h}$$

with $T_Q$ the percentage of the seeding flow flowing through the considered connection channel or segment, S the cross-section surface area of the connecting channel or segment taken perpendicular to its longitudinal fiber for the seeding flow, and h the height of the connecting channel or segment taken in the height direction.

It is noted that the distribution of the flow, and accordingly $T_Q$ values, may vary as a function of the flow rate for a given microfluidic device, due to fluid properties, such as when the fluid is a suspension of elongated particles or a shear-thinning fluid, or due to inertia effects, notably to inertia effects at channels intersections such as those occurring in so-called "Tesla valves". Throughout this document, the distribution of the flow, and accordingly $T_Q$ values, are estimated, for all assessments, by ignoring such effects, in particular by estimating the distribution of the flow based on numerical simulations of water flow at 37°C and at low flow rate where inertia effects are negligible, in particular a total seeding or harvest flow rate of 0.1 µL/s will be used for these estimations.

Thanks to the specific selection of the seeding shear indicators according to the invention, when seeding flow is applied through the input seeding channels, connection channels and the processing chamber, relatively higher shear rate upstream can detach, resuspend and move the particles toward the processing chamber, whereas in the processing chamber, relatively lower shear rate and velocity allows sedimentation of suspended particles and prevents displacement, detachment and resuspension of sedimented particles. The seeding shear indicator in the processing chamber, which is much lower than that in the input seeding channels and connection channels, ensures that a seeding flow at a seeding flow rate efficiently displacing particles in the input seeding channels and connection channels does not move too much, and preferably not at all, particles previously seeded in the processing chamber. In particular, in the case of a pulse-wise seeding flow, particles seeded during one pulse remain trapped and are not expelled during the next seeding pulses.

Such a configuration, combined with the presence of a single input seeding tree and a single output seeding tree that perfuse serially all segments of the processing chamber through connection channels, makes it possible to provide a seeding function with compensation of dispersion, leading to homogeneous seeding of the processing chamber, and suspension concentration, which is advantageously obtained without any filter or centrifugation machine which ordinarily result in supplementary costs, complexity and potential loss of performance or adverse effects on particles. In addition to the specific arrangement of the seeding channels and connection channels relative to the segments of the processing chamber, the microfluidic device according to the invention provides a harvest function with efficient and robust harvest capability, independently from the seeding function, so as to be able to induce relatively high shear rate within the processing chamber without requiring too high pressure.

It is noted that, in the invention, the most relevant shear rate is that occurring near the walls of the microfluidic device, and in particular near the bottom surface of the microfluidic device, since it is the value of the shear rate near these walls which indicates the magnitude of shear-related effects on particles positioned near the walls, and most notably is indicative of the forces susceptible to resuspend particles depending on the properties of the particles, properties of the walls and properties of the fluid. Thus, in the invention, estimates of the shear rate, unless otherwise specified, are calculated with the following formula:

$$Shear\ Rate = \frac{Q}{S * h}$$

where Q is the flow rate across a considered cross section taken perpendicular to the flow, S is the considered cross-section surface area, and h is the cross section average height taken in the height direction.

According to an advantageous feature, for the first segment of the processing chamber, in more than half of the input seeding channel volume of the input seeding tree linked to the segment, the ratio of the seeding shear indicator of the input seeding channels to the average seeding shear indicator of the first segment of the processing chamber is higher than 10, preferably higher than 20, more preferably higher than 40.

According to one embodiment, in the input seeding tree, the seeding shear indicator of the input seeding channels is substantially constant. Such a configuration for every input seeding channel results in an approximately constant shear rate in the input seeding channels. This makes it possible to select a flow rate that results in high enough shear rate to efficiently move particles toward the processing chamber during seeding, without important local peaks which would create lysis risks or local minima which would tend to trap particles.

In practice, it can be difficult to maintain the seeding shear indicator constant in the input seeding tree. In such cases, the range of values of the seeding shear indicator preferably covers less than 3 orders of magnitude, preferably less than 2 orders of magnitudes, and more preferably less than 1 order of magnitude.

According to one embodiment, each seeding tree of the microfluidic device is symmetric in terms of hydraulic resistance, i.e. the seeding channels between nodes of the same hierarchical level have the same hydraulic resistance for the same fluid at the same temperature, as can be numerically or analytically estimated. Such a symmetry in terms of hydraulic resistance improves the homogeneity of the seeding flow.

According to one embodiment, each seeding tree of the microfluidic device is symmetric in terms of geometry, i.e. all nodes of the seeding tree of the same hierarchical level, including the junctions with the processing chamber, are at the same channel path distance with respect to the tree root; and the cross section of the seeding channels at a given channel path distance from the tree root is the same for all channels. Such a geometry is a way of obtaining a seeding tree which is symmetric in terms of hydraulic resistance.

To further simplify the design of the microfluidic device, according to the invention, each seeding tree of the microfluidic device may additionally be designed to be, at least in part, a binary tree. This means that all nodes of the seeding tree of a given hierarchical level have two emerging channels. For optimized results, nodes of lower hierarchical levels, i.e. closer to the tree root, are binary with higher priority than the nodes of higher hierarchical levels, i.e. closer to the junctions with the processing chamber. The design is particularly simplified when each seeding tree is an entirely binary tree.

According to one embodiment, in the connection channels, the seeding shear indicator of the connection channels is substantially constant. Such a configuration for every connection channel results in an approximately constant shear rate in the connection channels. This makes it possible to select a flow rate that results in high enough shear rate to efficiently move particles from a segment to next segment during seeding, without important local peaks which would create lysis risks or local minima which would tend to trap particles.

According to one embodiment, the microfluidic device comprises blocking means configured to block selectively the input and output harvest channels when a seeding flow is applied to the processing chamber through the input seeding channels, connection channels and output seeding channels.

According to one embodiment, the microfluidic device comprises blocking means configured to block selectively the input seeding channels and output seeding channels when a harvest flow is applied to the processing chamber through the input and output harvest channels.

According to one embodiment, the microfluidic device comprises blocking means configured to block selectively the connection channels when a harvest flow is applied to the processing chamber through the input and output harvest channels.

The three preceding embodiments of blocking means may be combined so as to manage precisely desired flows and avoid parasitic flows in the microfluidic device.

Blocking means may comprise a displaced membrane capable of collapsing into a set of channels. In such cases channels preferably have a cross section favorable to the complete pinching of the channel inner volume by the membrane displacement. Such blocking means may be extended over a large fraction of channels to reduce volumes of harvest channels, fluidic connectors, connecting channels and seeding channels when not used and thus decrease reactant consumption. Such membrane-based blocking means may be actuated by a fluid on the other side of the membrane opposed to the channel.

In a particular configuration, activated connection channels may behave as blocking mean. Indeed, activated connection channels present different configurations with different thicknesses. In their undeformed state, activated connection channels have a thickness comparable to the thickness of the chamber. After deformation, activated connection channels may have thinner thickness, defining connection channels. Upon further deformation, activated connection channel may be completely pinched out, with almost null thickness, defining a blocking mean.

According to one feature, the pitch between adjacent junctions of the input seeding channels with the first segment of the processing chamber and the pitch between adjacent junctions of the output seeding channels with the second segment of the processing chamber is the same. Such a constant pitch on both sides of the serially connected segments contributes to a homogeneous seeding flow and homogeneous perfusion of the processing chamber.

According to one feature, the pitch between adjacent junctions of the connecting channels with the segments and the pitch between adjacent junctions of the input seeding channels with the first segment of the processing chamber is the same. Such a constant pitch on all sides of the serially connected segments contributes to a homogeneous seeding flow and homogeneous perfusion of the processing chamber.

According to one feature, connecting channels have all the same length. **In** this particular configuration, segments of processing chamber are parallel and seeding flow lines in all segments are similar, allowing for a better homogeneity of seeding.

According to another feature, in each seeding tree, the cross section of the seeding channels decreases with increasing channel path distance from the tree root. More precisely, the pitch between adjacent junctions of the seeding channels with the segment of the processing chamber and the cross section of individual seeding channels at a given channel path distance from the tree root are adjusted considering the flow rate splitting in the seeding channels of the seeding tree to result in a shear rate of approximately "n times" that occurring in the processing chamber where n, as mentioned above, is higher than 10, preferably higher than 20, more preferably higher than 40. **In** the case of binary seeding trees, the number of seeding channels of the binary trees, which is a power of two, is adjusted to reach the desired results.

According to one embodiment, for the first segment of the processing chamber, the ratio of the cross section of the first segment perpendicular to the transverse direction to the sum of the cross sections of the input seeding channels perfusing the first segment, *i.e.* closer to the junctions with the segment, is higher than 5, preferably higher than 10, more preferably higher than 15.

According to one embodiment, for all segments of the processing chamber, the ratio of the cross section of the segment perpendicular to the transverse direction to the sum of the cross sections of the connection channels perfusing the segment on one of its side is higher than 5, preferably higher than 10, more preferably higher than 15.

According to one embodiment, the seeding shear rate profile, obtained by estimating the shear rate during seeding flow in narrow cross sections perpendicular to the seeding flow of length dL at successive positions along the median longitudinal fiber L of the processing chamber, is characterized by a relative standard variation of less than 66%, preferably less than 33%, more preferably less than 10%, such profile being estimated with dL being constant, equal to less than 1% of the total length of the processing chamber median fiber L, and dL times the number of these cross-sections being equal to the total length of the processing chamber medial fiber L, in order to improve seeding performance and homogeneity.

According to one embodiment, the seeding volumetric profile, which is estimated similarly along the processing median fiber L by calculating, on the same successive cross sections, the ratio between the seeding flow rate crossing this cross section, and the local width of the processing chamber, estimated by the sum of the distances between this cross-section and the closest seeding tree or connection channels junctions with the processing chamber on both sides of this cross section, is characterized by a relative standard variation of less than 66%, preferably less than 33%, more preferably less than 10%, in order to improve seeding performance and in particular reduce the amount of particles lost during a seeding pulse.

According to one embodiment, the harvest shear rate profile, obtained by estimating the shear rate during harvest flow in cross sections perpendicular to the harvest flow along the processing chamber median fiber L, where such profile is obtained with at least 100 successive measurement cross sections homogeneously distributed along the entire length of the processing chamber segments perfused by the considered harvest flow, is characterized by a relative standard variation of less than 66%, preferably less than 33%, more preferably less than 10%, in order to improve harvest efficacy and homogeneity.

According to one embodiment, the total volume of the seeding trees is less than the total volume of the processing chamber, preferably less than 33% of the total volume of the processing chamber, more preferably less than 10% of the total volume of the processing chamber, in order improve the reactant use efficiency.

In an advantageous embodiment of the invention, the junction of each input seeding channel, output seeding channel and connection channel with the processing chamber is located in an upper part of the processing chamber in the height direction. Such a configuration takes advantage of particle inertia - when particle relative density is greater than the relative density of the surrounding fluid - during seeding to obtain a more homogeneous particle distribution in the processing chamber and in particular avoid that newly seeded particles travel near the bottom surface of the processing chamber during the seeding pulse.

According to one feature of the invention, for each segment of the processing chamber, the ratio of the width of the segment, taken in the transverse direction, to the height of the segment, taken in the height direction, is higher than 5, preferably higher than 10, more preferably higher than 20. In this way, each segment of the processing chamber is a shallow segment, where laminar flow leads to a convective replacement of most of the fluid contained in the segment. A draft angle, i.e. an angle between the side walls of the segment of the processing chamber and the direction perpendicular to the bottom surface of this segment, as well as a fillet at the bottom edges, i.e. rounded bottom edges, can also be added to further facilitate the complete renewal of the fluid contained in the segment.

It is noted that, if the distance between the bottom surface of the segment and the bottom surface of the seeding channels or connection channels is greater than the width of the segment, one or several stable whirlpools, representing a large part of the processing chamber segment volume, can occur down in the segment upon application of the seeding flow. This results in little or no convective exchange between the whirlpools and the seeding channels or connection channels, which can be found to increase the volume of reactants required to perform certain bioprocessing operations.

In order to make manufacturing of the microfluidic device easier and reduce the risk of leaks between channels, it is advantageous to have a minimal distance between parallel channels. For example, a typical minimal distance between parallel channels is 1 mm, while accurate manufacturing methods may allow to reduce this minimal distance, e.g. to 500 $\mu$m or less. It can be found advantageous to manufacture parts of the microfluidic device by injection molding or hot embossing of polymer materials, such as for example polystyrene, cyclic olefin polymers or copolymers, notably to achieve low cost per unit. In such cases, demolding is facilitated, and resulting accuracy is improved, by applying a minimal draft angle as mentioned above.

According to one embodiment, the processing chamber and the channels of the microfluidic device all share the same top surface plane, in order to allow closing the microfluidic device with a flat sheet of material, such as polymer, which makes manufacturing easier. In a particular embodiment, the microfluidic device may comprise an injection-molded or hot-embossed bottom part made of a polymer material and a top part, such as a flat sheet, made of the same or a different polymer material.

Mold fabrication may additionally be facilitated by rounding inner edges of channel bends of the microfluidic device with a minimal radius of curvature, of the order of 50 $\mu$m or 100 $\mu$m. Advantageously, outer edges of channel bends may also be rounded with a minimal curvature, notably to regularize the flow in the bends.

According to one embodiment, the microfluidic device is extremely clean in terms of chemical residues, dust and particles, endotoxins and other biological contaminants, which may be achieved by adjusting the manufacturing methods and/or by implementing post-processing steps involving for example sonication, irradiation, washing, autoclaving, etc. In a preferred embodiment, the microfluidic device is made of medical grade materials. In a preferred embodiment, the parts of the microfluidic device that are made of polymer are free of plasticizer.

According to one embodiment, the microfluidic device is sterilized, for example through gamma ray irradiation, within a packaging such as a sealed polymer bag. This reduces the risk of contamination when using the microfluidic device.

According to one embodiment, the microfluidic device is a single-use device, so as to reduce cross-contamination and/or interference between successive processes.

According to one embodiment, outer connectors or connection ports of the microfluidic device, making it possible to fluidly connect the microfluidic device with other systems, have an internal diameter less than or equal to 2 mm, preferably less than or equal to 1 mm. Such a configuration reduces so-called "dead volumes" and improves bioproduction efficiency.

In order to reduce particle losses during seeding, the processing chamber is advantageously seeded with seeding flow pulses, representing a volume lower than that of the processing chamber, and a seeding pause between the seeding flow pulses, corresponding to no or very limited flow applied so as to let suspended particles sediment in the segments of the processing chamber. The flow rate during a seeding pulse is selected, depending on the design of the microfluidic device, to be small enough not to result in a shear rate in the segment of the processing chamber that would result in resuspension or large displacement of sedimented particles, and large enough so that the input particle suspension does not sediment too fast during the seeding flow pulse, in which case particles would accumulate toward the input seeding side of the segments of the processing chamber, and also so that the upstream shear rate is sufficient to efficiently move particles toward the segment of the processing chamber.

Alternatively, seeding flow pulses of a volume equal or greater to 50% of the processing chamber volume, susceptible to result in particles in output seeding channels after the pulse, is followed by a seeding pause, then a reverted (from output seeding tree towards input seeding tree) seeding flow pulse meant to bring particles possibly deposited in the output seeding channels back in the processing chamber, then another seeding pause.

In some embodiments where blocking means result in fluid being expelled from blocked channels into neighboring channels or cavities, these blocking means may advantageously be activated during seeding pauses in order to minimize particle deposition in seeding channels and connecting channels. This is in particular true when blocking means comprise a displaced membrane pinching the blocked channels.

According to an advantageous feature, the pitch between adjacent junctions of the seeding channels with the first segment of the processing chamber is less than the width of the first segment of the processing chamber taken in the transverse direction, preferably less than 1/2 of the width of the first segment of the processing chamber, more preferably less than 1/3 of the width of the first segment of the processing chamber. Similarly, the pitch between adjacent junctions of the connection channels with a segment is less than the width of the segment of the processing chamber taken in the transverse direction, preferably less than 1/2 of the width of the segment of the processing chamber, more preferably less than 1/3 of the width of the segment of the processing chamber. **In** the case where two segments are connected by one connection channel only, equivalent effects are obtained with the width of the connection channel being as close as possible to the length of the joined segments. **In** this way, a more homogeneous seeding flow occurs within the segments of the processing chamber.

According to one feature of the invention, the junction of each input and output seeding channel with the segments of the processing chamber is chamfered. Such chamfers at the junction between the seeding channels and the processing chamber make it possible to have an increased length of the segment of the processing chamber, resulting in higher capacity of the microfluidic device, without leading to heterogeneous seeding flow. Preferably, each chamfer has an angle of less than 45° relative to the longitudinal direction of the seeding channel at its junction with the segment of the processing chamber, so as to provide homogeneous seeding flow.

According to one embodiment, the seeding and harvest channels are configured such that, upon application of a harvest flow in the processing chamber, the absolute flow rate in each seeding channel and each connection channel is less than 10%, preferably less than 5%, more preferably less than 2.5%, of the sum of the flow rates of the harvest flow applied at the junction of each input harvest channel with the processing chamber. The reduction of this percentage, which can be described as a shunt flow during a harvest flow, helps reduce the risk of particles being lost in the seeding channels or connection channels during the harvest, which in turn increases the performance of the harvest.

More generally, according to the invention, the seeding channels and connection channels are characterized by lower cross sections and higher shear rate than those of the processing chamber during a seeding flow, whereas the harvest channels are characterized by cross sections and shear rate similar to those of the processing chamber during a harvest flow.

In an advantageous manner, the height of the processing chamber, taken in the height direction, is comprised between $50\mu m$ and $300\mu m$. This range of height is found to be compatible with moderate resistance to the flow. It is noted that the bottom surface of the processing chamber is, in most cases, representative of the production capacity of the microfluidic device, whereas the height of the processing chamber is representative of the fluid volume per unit of production capacity. Thus, this range of height is found to advantageously minimize the amount of reactant required to perform a bioproduction operation relative to the number of processed particles. Preferably, the height of the processing chamber is constant. More generally, the processing chamber is designed to have a low impact on the flow distribution, by exhibiting low hydraulic resistance, minimal size and number of obstacles to the flow.

In an advantageous manner, the surface of the processing chamber, perpendicular to the height direction, is greater than 0.25 cm$^2$, preferably greater than 1 cm$^2$, more preferably greater than 4 cm$^2$, more preferably greater than 10 cm$^2$, more preferably greater than 16 cm$^2$, more preferably greater than 20 cm$^2$, more preferably greater than 30 cm$^2$. In other words, the surface of processing chamber may represent more than 45% of the whole surface of the microfluidic device, even more than 50%, 55%, 60% or 70%. Such a high surface area ensures sufficient particle handling capacity for bioproduction.

According to one feature of the invention, at least one of the upper wall and the lower wall of each segment of the processing chamber, in the height direction, is transparent to an imaging wavelength of a device for imaging across the wall of the microfluidic device. This allows for clear imaging across the wall of the microfluidic device, so that process monitoring can be performed. The imaging wavelength may be comprised in the visible light, infrared and/or ultraviolet spectrum. According to one embodiment, the upper wall and/or the lower wall of the segment of the processing chamber are planar and parallel in order to facilitate focus and imaging. It is noted that the planarity of the surfaces does not preclude from the presence of microstructures providing, for example, particle adherence features. When such microstructures are present, their dimensions are preferably either much smaller or much greater than the imaging wavelength.

According to one feature of the invention, a bottom surface of the processing chamber is provided with a particle-adhesive coating, such as adhesion peptides, ECM molecules or fragments, antibodies, nanobodies, cell membrane anchoring molecules. Similarly, the seeding channels and connection channels may be provided with a coating to reduce the adherence of the particles, such as a hydrophilic coating based on e.g. albumin such as Bovine Serum Albumin (BSA), polyhydroxyethylmethacrylate (pHEMA), poly-L-lysin (PLL), polyethyleneglycol (PEG), carboxybetain, agar, cellulose, chitosan, or their polymers or copolymers. Alternatively, surface microstructures and/or roughness can be chosen for similar purposes in the processing chamber, so that the shear rate near the bottom surface of the processing chamber traps particles, and in the seeding channels and connection channels, so that the shear rate near the bottom surface of the seeding channels and connection channels is sufficiently strong to compensate for particle adhesion and provide particle movement.

According to one embodiment, the microfluidic device comprises fluidic connectors between segments of the processing chamber. These fluidic connectors are configured to allow the harvest flow through all the segments of the processing chamber serially.

In this embodiment, the cross section of the fluidic connectors is preferably adapted to create a moderate resistance to the flow while representing a minimal volume, as this volume is often a "dead volume" in terms of production capacity of the device. For example, the cross section of each fluidic connector can be in the order of that of the processing chamber perpendicular to its longitudinal axis, or in the order of half of this cross section.

Beyond a certain size of the microfluidic device (or chip) where hydraulic resistance becomes too high to perform seeding or harvest efficiently, other arrangements of the segments of the processing chamber may be considered, such as a configuration where several segments and attached channels are connected in parallel in the same plane, or a configuration where several segments and attached channels are stacked vertically, or a configuration where several segments are distributed radially, or a configuration where several segments are disposed along a curve. Yet, compared to the configuration where segments of the processing chamber are connected in a serpentine shape, these configurations multiply the number of fluidic connections, in particular of connection channels of different sizes and shapes making it difficult to design an adequate seeding flow; and, in the case of the stacked configuration, it makes it more complicated to perform optical microscopy analysis of the content of the processing chamber.

According to one embodiment, the microfluidic device comprises a gas permeable membrane which forms at least part of the top surface and/or the bottom surface of the processing chamber. The purpose of this membrane is to provide gas exchange (the membrane may also be called a gas exchange membrane). To this end, a gas exchange medium containing a given concentration of a gas in a gas phase or dissolved in a liquid is positioned on one side of the gas permeable membrane which is oriented outwardly relative to the processing chamber. This embodiment is interesting, e.g. for the handling of particles that are sensitive to dissolved gas concentrations or for the handling of particles capable of chemically transforming dissolved gases such as living animal cells.

According to one embodiment, the microfluidic device comprises a polymer part including the processing chamber and the seeding, harvest and connection channels formed therein, the polymer part being sealed by means of a gas permeable membrane. The polymer part may be made of an elastomeric or rigid polymer, for example a cyclic olefin copolymer (COC). In this embodiment, the gas permeable membrane forming the top surface or the bottom surface of the processing chamber may also form the top surface or the bottom surface of the seeding, harvest and connection channels of the microfluidic device. The polymer part may be manufactured, for example, by injection molding or hot embossing. The gas permeable membrane may advantageously be made of a transparent material, such as, for example, peroxide or platinum cured silicone, so as to preserve imaging capabilities. The polymer part and the gas permeable membrane may be assembled using any conventional assembly means known in the art. In particular, the gas permeable membrane may be deformable and used locally as activated connection channel or blocking mean.

If the gas permeable membrane forms the top surface of the processing chamber, the junction of each input seeding channel, output seeding channel and connection channel with the processing chamber may be located in a lower part of the processing chamber in the height direction. This configuration allows for a simple setup of gas exchanges with processing chamber without modifying geometry of the input seeding channel, output seeding channel and connection channel when gas permeable membrane is reported on the polymer part.

To the contrary, if the gas permeable membrane forms the top surface of the processing chamber, the junction of each input seeding channel, output seeding channel and connection channel with the processing chamber may be located in an upper part of the processing chamber in the height direction. With a deformable gas permeable membrane, this configuration allows for a simple definition of blocking means, pushing on the membrane to pinch out connection channels.

According to one feature, the gas permeable membrane is designed with a thickness sufficient to hold its shape and to hold pressure differences across its height. However, an excessive thickness of the gas permeable membrane should also be avoided, in particular because of the induced limitation of gas exchange and the risk of collapse of the membrane in the processing chamber due to its elastic deformation following, e.g. application of pressure externally to the microfluidic device to avoid leaks. By way of example, a gas exchange membrane, e.g. made of silicone, may have a thickness between 10 $\mu$m and 2 mm, preferably between 15 $\mu$m and 1 mm, more preferably between 20 $\mu$m and 250 $\mu$m depending on its gas diffusion and mechanical properties.

When a gas permeable membrane forms at least part of the bottom surface of the processing chamber, the particles may lie on the gas permeable membrane after seeding, thus allowing better control of the dissolved gas concentrations due to a reduced distance between the particles and the outer side of the gas permeable membrane, oriented outwardly relative to the processing chamber. It is also possible to obtain specific biochemical properties of the bottom surface of the processing chamber, such as selective adhesion properties, by using a gas permeable membrane that has been chemically modified, and in particular a chemically coated, e.g. using silanes which can be used to bind antibodies or antibody fractions to the bottom surface of the processing chamber.

When a gas permeable membrane forms at least part of the top surface and/or the bottom surface of the processing chamber, it may be advantageous to equip the microfluidic device with a "gas chip" configured to control the flow or diffusion in the gas exchange medium on the outer side of the gas permeable membrane, and also optionally to provide mechanical support on the outer side of the gas permeable membrane. The gas chip is advantageously made of a transparent material so as to preserve imaging capabilities of the device. Because the gas chip is not in direct contact with the processed particles, a wide range of materials may be used. The gas chip may comprise interconnected channels or chambers having their bottom or top surface formed by the gas permeable membrane.

In a preferred embodiment, the gas chip comprises a chamber with an array of pillars or an array of channels positioned facing the processing chamber, so as to limit the deformation of the gas permeable membrane outwardly relative to the processing chamber. In this case, the chamber of the gas chip and the array of pillars or the array of channels are preferably arranged so as to minimally impact the imaging of the content of the processing chamber, e.g. by reducing the height of the pillars or channels and increasing their density. Such an arrangement makes it possible to maintain the geometry of the microfluidic device, in particular when the pressure in the microfluidic device is higher than that in the gas exchange medium, even in the absence of strong chemical bonds between the gas permeable membrane and the other parts of the device, which is often the case, in particular due to the difficulty to establish strong chemical bonds between plastics and silicones. The gas exchange medium can also be optimized so as to reduce interference with imaging. By way of example, the gas exchange medium may be a liquid substantially transparent across the chosen imaging spectrum, e.g. the visible spectrum, and with a refraction index matching that of the gas chip material across the chosen imaging spectrum.

The gas chip also preferably comprises input and output channels for the renewal of the gas exchange medium, which may be connected to an external module configured to renew the gas exchange medium or to adjust its gas concentration. The input and output channels of the gas chip are preferably arranged so as to avoid the regions facing the input and output seeding channels of the microfluidic device, *e.g.* by following the path of the input and output harvest channels. Additional channels of the gas chip may be arranged facing fluidic connectors of the microfluidic device connecting the processing chambers for the harvest flow. Such arrangements allow a better controlled and a more homogenous seeding. The gas chip and the microfluidic device including the gas permeable membrane may be assembled using any conventional assembly means known in the art, such as covalent bonding, adhesives, mechanical clamping, magnetic clamping, etc.

Another subject of the invention is a method for processing particles, in particular cells, using a microfluidic device as described above, the method comprising:

- a step of seeding the processing chamber with particles by applying a seeding flow to the processing chamber through the input seeding channels, connection channels and output seeding channels, while the input and output harvest channels are blocked,
- a step of collecting particles from the processing chamber by applying a harvest flow to the processing chamber through the input and output harvest channels, while the input seeding channels, connection channels and output seeding channels are blocked.

In a particular embodiment of this method, the step of seeding the processing chamber is done while fluidic connectors are blocked.

In the section above and the rest of the document, when a set of channels is said to be blocked, what is meant is that, at a minimum, the flow between the channels of this set and the outside of the microfluidic device, or any other device, is inhibited, for example by the means of one or several valves positioned in the microfluidic device or on a flow line connected to it. While the considered set of channels is blocked, some flow could occur between this set of channels and a segment of processing chamber to which it is connected. However, when this expression is used, it is often preferable that any flow within this set of channels is inhibited.

The application of the harvest flow to the processing chamber through the harvest channels, while the seeding channels are blocked, provides at relatively low hydraulic resistance a sufficient shear rate to recollect the particles from the processing chamber very efficiently.

According to one embodiment, the flow in the seeding channels may be prevented, prior to applying the harvest flow, by infusing occluding material in the seeding channels, such as beads of appropriate dimensions. This is found to be particularly advantageous in cases where the flow in the seeding channels during the harvest flow is high and interferes with the harvest.

According to one embodiment, the step of seeding the processing chamber is carried out by applying successive pulses of seeding flow separated by a resting time. In this way, particles in suspension are infused pulse-wise by a flow through the segments of the processing chamber and connection channels from the input seeding channels to the output seeding channels while harvest channels - and, as case may be, fluidic connectors - are blocked, which results in suspended particles travelling down the input seeding channels into the segments of the processing chamber and connection channels, settling and remaining trapped in the processing chamber. Optionally each seeding flow pulse, *i.e.* from input to output seeding channels, may be separated by a smaller volume reverted seeding flow pulse, *i.e.* from output to input seeding channels, flanked temporally by seeding pauses. This option allows limiting particle losses downstream of the processing chambers during seeding as well as improving the seeding homogeneity by compensating for the non-flat velocity profile of the flow.

In an improved embodiment, each pair of seeding pulse is separated by a reverted-flow seeding pulse flanked by two seeding pauses, with seeding pulses of greater ratio R and reverted-flow seeding pulses of smaller ratio R. Within the invention, the ratio R of a seeding flow represents the volume of the seeding flow divided by the volume of the processing chamber, *i.e.* the sum of volumes of all segment arranged serially. The ratio R of direct seeding pulses is noted $R\_dir$ and that of counter-flow pulses $R\_rev$. Reverted-flow seeding pulses are applied with opposite flow-rate compared to ordinary (direct) seeding pulses, *i.e.* reverted flow is from the output to the input seeding channels. Absolute value of the flow rate of reverted-flow seeding pulses is chosen similarly to that of direct seeding pulses. Reverted-flow seeding pulses added between normal seeding pulses, all separated by seeding pauses, allow using higher $R\_dir$ direct seeding pulses leading to better dispatching of cells within processing chamber segments but usually with a number of cells residing in output seeding channels after the pulse. The reverted flow seeding pulse after the pause then allows recovering these cells and depositing them in the segments of the chamber closest to output seeding channels where usually fewer cells are seeded, further improving seeding homogeneity. Pauses between each seeding pulse (direct or reverted flow) allow for cells deposited in segments of the processing chamber not to be displaced significantly by following pulses. $R\_dir$ may range from 100% to 250%, and $R\_rev$ may range from 20% to 170%. In a particular embodiment, the effective ratio R for a direct seeding flow followed by a reverted seeding flow is $R=R\_dir - R\_rev$ and may range from 70% to 110%.

According to one embodiment, the step of collecting particles from the processing chamber is carried out by applying successively different flow rates of the harvest flow while the seeding channels are blocked. The different flow rates of the harvest flow are advantageously adapted to collect particles having different magnitudes of adherence to the surfaces of the processing chamber and/or different flow induced forces to which they are submitted and/or different aggregation states with other particles.

Features and advantages of the invention will become apparent from the following description of embodiments of a microfluidic device and a method for processing particles according to the invention, this description being given merely by way of example and with reference to the appended drawings.

## BRIEF DESCRIPTION OF THE DRAWINGS

**Figure 1** is an upper view of a microfluidic device according to a first embodiment of the invention,

**Figure 2** is a cross section along the line II-II of Figure 1,

**Figure 3** is a view similar to Figure 1 for a second embodiment of a microfluidic device according to the invention,

**Figure 4** are views similar to Figure 1 for a third embodiment of a microfluidic device according to the invention, in two configurations: Figure **4a** with permanent connection channels and Figure **4b** with activated connection channels.

**Figure 5** is a view similar to Figure 1 for a fourth embodiment of a microfluidic device according to the invention,

**Figures 6** and **7** are cross sections of two variants of a microfluidic device according to a fifth embodiment of the invention,

**Figures 8a, 8b, 8c** are cross sections of a microfluidic device equipped with a gas chip according to three variants of a sixth embodiment of the invention, and

**Figures 9a, 9b, 9c** are upper views of the gas chip in each of the variants of Figures 7a, 7b, 7c, respectively.

## DETAILED DESCRIPTION

*First embodiment - identical segments*

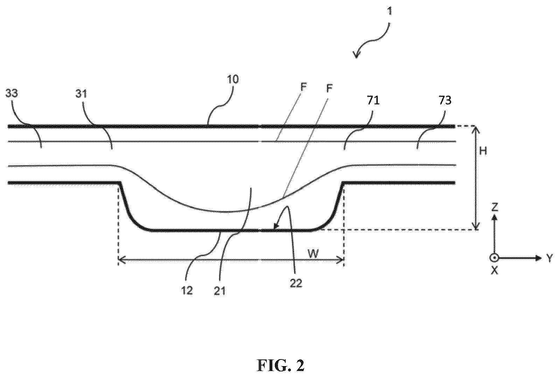

In the first embodiment shown in Figures 1 and 2, the microfluidic device 1 comprises a processing chamber 2 comprising two segments 21 extending along a longitudinal axis X. In a first variant of this embodiment, the two segments 21 are identical. The microfluidic device 1 also comprises a single input seeding tree 3, defining a plurality of input seeding channels 33, and a single output seeding tree 4, defining a plurality of output seeding channels 43. The junctions 31, 41 of the input seeding channels 33 and the output seeding channels 43 with the first and second segments 21 of the processing chamber are respectively distributed along one side of the first and second segments 21. The microfluidic device 1 also comprises connection channels 73, each connection channel having junctions 71 with both segments. Both segments are arranged serially with respect of input seeding tree 3 and output seeding tree 4.

More specifically, in this particular embodiment, the input seeding tree 3 and the output seeding tree 4 are binary trees which are arranged facing each other on both sides of segments 21 arranged serially. The input seeding channels 33 and the output seeding channels 43 are configured to define a seeding flow in a direction Y transverse to the longitudinal direction X of the segments 21. Each of the seeding binary trees 3, 4 is symmetric in terms of hydraulic resistance, which is advantageous to ensure a homogeneous seeding flow.

The pitch p between adjacent junctions 31 of the input seeding channels 33 with the first segment 21, between adjacent junctions 41 of the output seeding channels 43 with the second segment 21 and between adjacent junctions 71 of the connection channels 73 with both segments 21 is the same, which also contributes to a homogeneous seeding flow and homogeneous perfusion of the processing chamber 2. As can be seen in Figure 2, the junctions 31, 71 of each input seeding channel 33 and connection channel 73 with the first segment 21 is located in an upper part of the processing chamber 2 in the height direction Z. Output seeding channels 43 are in the same configuration (not shown). Such a configuration takes advantage of inertia, under appropriate seeding flow rate, to more homogeneously distribute particles within the processing chamber after a seeding pulse. The seeding pause can then be used to allow the particles to sediment on the bottom surface 22 of the processing chamber. Oppositely, under lower flow rates, under the effect of gravity, particles can be found to follow the lower streamline F, as shown in Figure 2, which is less advantageous notably in terms of seeding homogeneity.

In an advantageous manner, the bottom surface 22 of the segment 21 is provided with a particle-adhesive coating, such as adhesion peptides, ECM molecules or fragments, antibodies, nanobodies, cell membrane anchoring molecules. For example, segment 21 bottom surface may be coated by a silane-PEG-biotin or a PLL-PEG-biotin, biotin-streptavidin linkage may then be used to graft specific biological molecules. Conversely, the input seeding channels 33 and connection channels 73 are advantageously provided with a coating to reduce the adherence of the particles, such as a hydrophilic coating based on BSA, pHEMA, PLL, PEG, carboxybetain, agar, cellulose, chitosan, or their polymers or copolymers. Coating of microfluidic chips by incubation of a liquid solution of coating reactants is found to be greatly improved, in particular in terms of homogeneity, by applying flow of the liquid solution during incubation, and in particular repeated back and forth flow during the incubation. Additionally, solid particles small enough not to clog channels and easily washed after coating may be added to coating solutions to favor mixing by flow-induced rotation.

In this embodiment, the ratios *Rs_input, Rs_output* and *Rs_connect* are higher than 50, whereas the the ratios *Rh_input* and *Rh_output* are lower than 20.

In this embodiment, the ratio of the cross section $S_{21}$ of the segment 21 of the processing chamber perpendicular to the transverse direction Y to the sum of the cross sections $S_{31}$ of the input seeding channels 33 at the junction 31 with the processing chamber 2 is higher than 15. In addition, the ratio of the width W of the segment 21 of the processing chamber, taken in the transverse direction Y, to the height H of the segment 21, taken in the height direction Z, is higher than 20. In this way, the segment 21 of the processing chamber is a shallow segment, where laminar flow leads to a convective replacement of most of the fluid contained in the segment. The angle to the side walls of the segment 21 of the processing chamber further facilitates the complete renewal of the fluid contained in the segment 21.

The microfluidic device 1 also comprises two input harvest channels 53 and two output harvest channels 63 configured to define serially a harvest flow in the longitudinal direction X of the segments 21 of the processing chamber 2. As can be seen in Figure 1, the junctions 51, 61 of the input harvest channels 53 and of the output harvest channels 63 with the segments of the processing chamber 2 are chamfered, which is favorable for the homogeneity of the harvest flow. The seeding, harvest and connection channels 33, 43, 53, 63, 73 are configured such that, upon application of a harvest flow in the segment 21 of the processing chamber, the absolute flow rate in each seeding channel 33, 43 or connection channel 73 is less than 2.5% of the flow rate of the harvest flow applied at the junction 51 of the input harvest channel 53 with the segments 21 of the processing chamber 2.

In this embodiment, the input seeding tree 3 has, in more than half of its channel volume, a ratio of the seeding shear rate indicator SSI of the input seeding channels 33 to the average seeding shear rate indicator SSI of the segment 21 of the processing chamber which is higher than 20, where the seeding shear rate indicator SSI of a channel or a segment is defined by:

$$SSI = \frac{T_Q}{S * h} = \frac{\dfrac{Q}{Qtot}}{S * h}$$

with $T_Q$ the percentage of the seeding flow flowing through the considered channel or segment, which is equal to the ratio of the seeding flow rate Q in the considered channel or segment to the total seeding flow rate $Qtot$, $S$ the cross section of the channel or segment taken perpendicular to its longitudinal fiber for the seeding flow, and h the height of the channel or segment taken in the height direction Z.

In a non-limiting example of the first embodiment:

- the segments 21 of the processing chamber 2 are identical and have a height H of 100μm and a width W of 5 mm,

- the cross section $S_{31}$ of the seeding channels 33, 43 and connection channels 73 at the junction 31, 41, 71 with the segments 21 of the processing chamber 2 is 100μm width by 50μm height,
- the pitch p between adjacent junctions 31 of the input seeding channels 33, the pitch p between adjacent junctions 41 of the output seeding channels 43 and the pitch p between adjacent junctions 71 of the connection channels 73 is constant and equal to 1.25 mm,
- the surface of the processing chamber 2, perpendicular to the height direction Z, is greater than 0.25 cm².

In this embodiment, the upper wall 10 and the lower wall 12 of the processing chamber 2, in the height direction Z, are transparent to the visible, near IR and near UV wavelengths, for example with the upper wall 10 or bottom wall 12 being made of cyclic olefin copolymer (COC). In this way, the bioprocess can be monitored by imaging across the walls of the microfluidic device 1. In this embodiment, the upper wall 10 and the lower wall 12 of the segments 21 of the processing chamber 2 are planar and parallel, which facilitates focus and imaging.

*First embodiment - different segments*

In this variant of first embodiment, segments 21 are different.

Hereafter, the first segment 21 corresponds to the segment connected to input seeding tree via junctions 31, while the second segment 21 corresponds to the segment connected to output seeding tree via junctions 41.

More specifically, first and second segment may differ by their height and/or surface coating in relationship with cell adherence.

In an embodiment, the height of first segment is in the range of 50 μm to 100 μm while the height of the second segment is in the range of 100 μm to 300 μm.

In an embodiment, first segment is coated to be anti-adherent (cells do not adhere to the surface) while second segment is coated to be adherent (cells do adhere to the surface).

In a non-limiting example, first segment may be thinner (height of 75 μm) and coated to be anti-adhesive (Poly-ethylene glycol coating) while second segment may be thicker (height 150 μm) and coated to be adhesive (Poly-L-Lysine coating). This configuration allows to bring cells into first segment 21 with the harvesting flow mode. Adhesion of cells is prevented by an anti-adherent coating. Then cells may be displaced from said first segment to said second segment with the seeding flow mode. In said second segment, cells sediment in the bottom of the segment and finally adhere to the surface. Seeding process is thus improved. Indeed, this allows to use a higher flow rate to bring cells in the first segment, which reduces upstream losses of cells in typical settings.

The cells are then easily displaced into the second segment using a smaller flowrate which creates less risk of displacing cells that would already reside in the second segment. Overall, this allows decoupling the input flow rate of the cell suspension from upstream volumes and the seeding flow rate of the cell suspension in the second segment to choose values which are optimal for both aspects. Additionally, it allows to use optical monitoring of the first segment prior to displacing cells in the second segment to check their quality before seeding the second segment.

*Second embodiment*

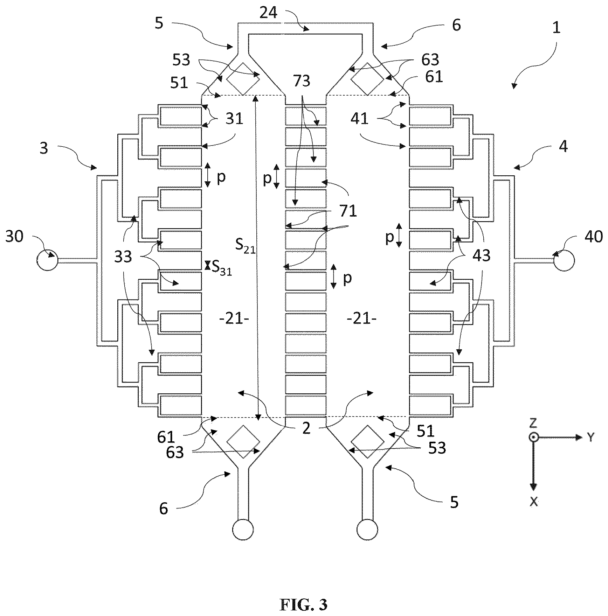

In the second embodiment shown in figure 3, elements similar to those of the first embodiment bear identical references. The microfluidic device 1 of the second embodiment differs from the first embodiment in that the single input harvest channels 53 and the single output harvest channels 63 are replaced by input and output harvest binary trees 5 and 6 having chamfered junctions 51, 61 with the segments 21 of the processing chamber 2. Compared to the first embodiment, the harvest trees 5 and 6 provide a more homogeneous harvest flow. It is noted that, in this second embodiment, the harvest trees 5, 6 and the seeding trees 3, 4 are not interchangeable. Indeed, the harvest flow provides, with a given flow rate, a much higher shear rate within the processing chamber than if the same flow rate was used to perfuse the microfluidic device with a seeding flow.

In this embodiment, the ratios *Rs_input, Rs_output* and *Rs_connect* are higher than 50, whereas the the ratios *Rh_input* and *Rh_output* are lower than 20.

*Third embodiment*

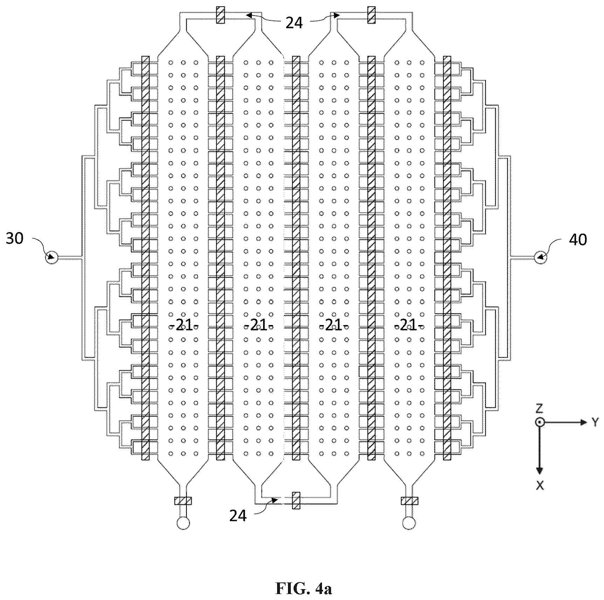

In the third embodiment shown in figure 4, elements similar to those of the first embodiment bear identical references. The microfluidic device 1 of the third embodiment differs from the first embodiment in that the processing chamber 2 includes four segments 21 that are connected by fluidic connectors 24, allowing for harvest flow through all the segments serially. In this third embodiment, the microfluidic device 1 comprises a single input seeding tree 3 in junction with the first segment and a single output seeding tree 4 in junction with the fourth segment and connection channels between first and second segments, second and third segments, third and fourth segments, allowing for a seeding flow through all the segments serially. This configuration is advantageous both for the compacity of the microfluidic device and for the homogeneity of the seeding flow throughout the processing chamber 2. The setup of fluidic connectors 24 in serpentine configuration makes it possible to optimize both the capacity of the microfluidic device 1 and its size, with a relatively simple design and a relatively simple manufacturing process since there is no need for a second channel layer.

In figure **4a,** blocking means (represented in hatched blocks) are disposed on all seeding channels, permanent connection channels and harvest channels. Opening and closing of these blocking means lowers parasitic flows in harvest channels during seeding flow and vice-versa.

In figure **4b,** blocking means (represented in hatched blocks) are disposed on all seeding channels and harvest channels. Connection channels are here activated connection channels. 3 activated connection channels 81 are arranged through the whole processing chamber, defining four elongate segments. When pinched out, the activated connection channels define 3 thin and large (as large as segments' length) connection channels. Upon further deformation, activated connection channel may be completely pinched out, with almost null thickness, defining a blocking mean.

In this embodiment, pillars (represented by regularly disposed circles) are added in segments between upper wall 10 and the lower wall 12 of the processing chamber. These pillars improve mechanical resistance and limit deformation of the microfluidic device upon pressure variations during operations.

In this embodiment, the ratios *Rs_input, Rs_output* and *Rs_connect* are higher than 50, whereas the the ratios *Rh_input* and *Rh_output* are lower than 20.

*Fourth embodiment*

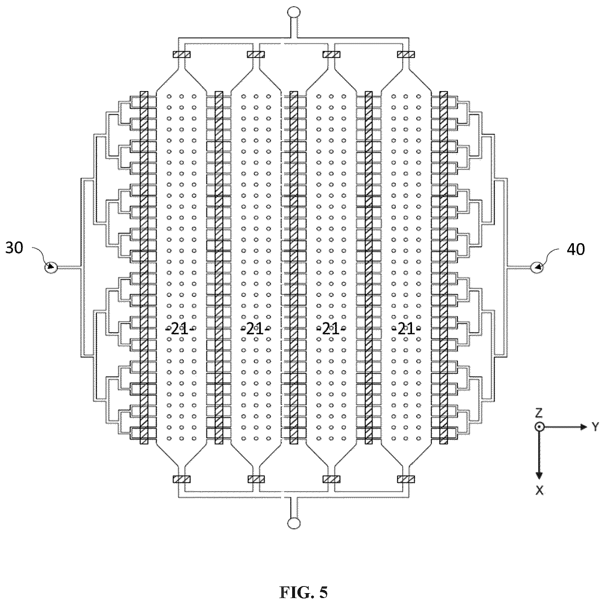

In the fourth embodiment shown in figure 5, elements similar to those of the first embodiment bear identical references. The microfluidic device 1 of the fourth embodiment differs from the third embodiment in that the four segments 21 of the processing chamber are in junction with a single input harvest tree and a single output harvest tree. In this embodiment, the harvest flow is done in parallel. By proper use of blocking means, it is possible to define a harvest flow on some of the segments 21 of the processing chamber 2 and not on all segments 21 simultaneously.

In this embodiment, the ratios *Rs_input, Rs_output* and *Rs_connect* are higher than 50, whereas the the ratios *Rh_input* and *Rh_output* are lower than 20.

*Fifth embodiment*

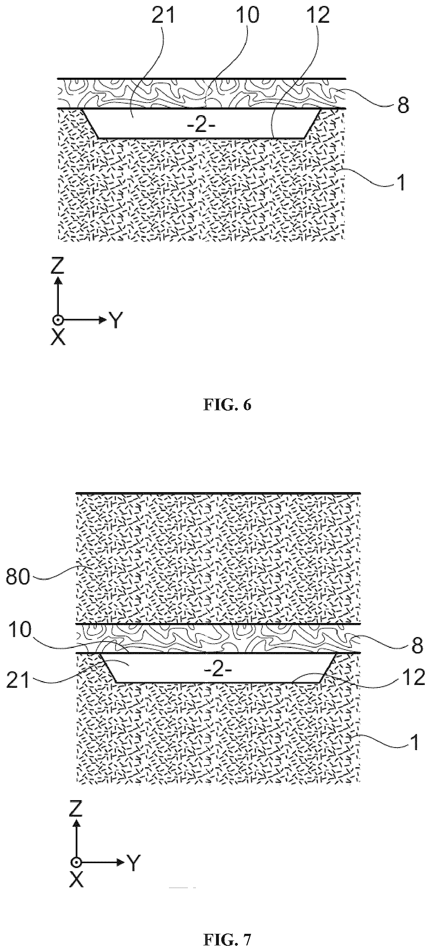

In the fifth embodiment shown in figures 6 and 7, elements similar to those of the fourth embodiment bear identical references. The microfluidic device 1 of the fifth embodiment differs from the fourth embodiment in that all channels and segments of the device share a common bottom surface formed by a gas permeable membrane 8 made of silicone and having a constant thickness between 50 μm and 2 mm. As a variant, the gas permeable membrane 8 may be made of any other material compatible with biomedical use, and preferably transparent. The remaining of the microfluidic device is formed by a polymer part, for example made of polystyrene (PS) or of a cyclic olefin copolymer (COC).

Figure 6 shows a configuration where the gas permeable membrane 8 forms the upper part of the microfluidic device 1, whereas figure 7 shows a configuration where the gas permeable membrane 8 is covered with an additional rigid plate 80. The additional rigid plate 80 makes it possible, during flow operations, to limit the deformation of the gas permeable membrane 8. Advantageously, the rigid plate 80 is positioned above the gas permeable membrane 8 for the duration of the flow operations and an average pressure equal to or greater than the perfusion pressure is applied on the rigid plate 80 using any suitable means, in particular mechanical means. In order to allow gas exchange across the gas permeable membrane 8 outside of flow operations, the rigid plate 80 is preferably removed after flow operations.

*Sixth embodiment*

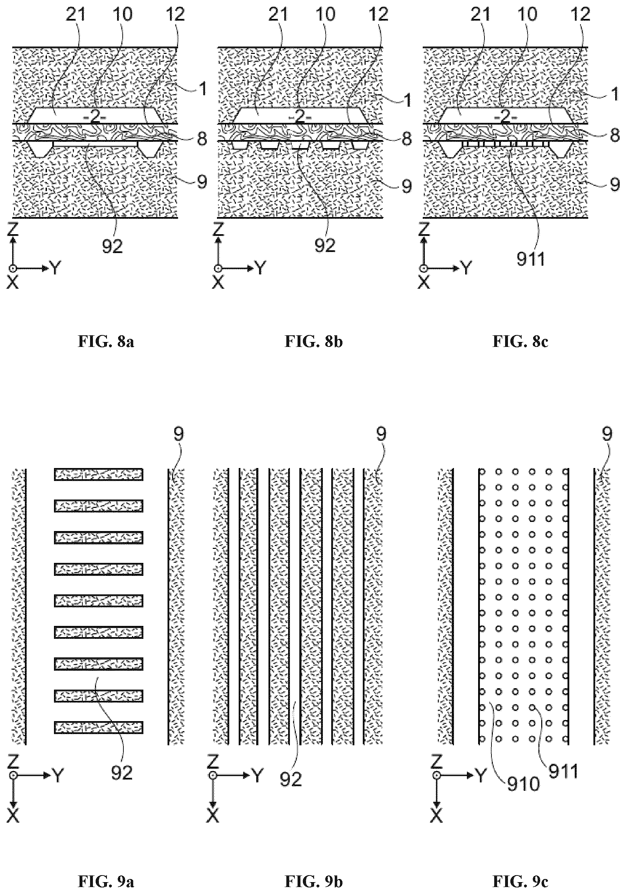

In the sixth embodiment shown in figures 8a, 8b, 8c and 9a, 9b, 9c, elements similar to those of the fifth embodiment bear identical references. The microfluidic device 1 of the sixth embodiment differs from the fifth embodiment in that it further comprises a gas chip 9 on the outer side of the gas permeable membrane 8, oriented outwardly relative to the segment 21 of the processing chamber, configured to control the flow or diffusion of gas. The gas chip 9 is preferably made of a rigid and transparent polymer material such as polystyrene (PS) or a cyclic olefin copolymer (COC). The gas chip 9 is closed by the gas permeable membrane 8 and comprises, positioned facing the processing chamber, a chamber 910 provided with an array of channels 92 as shown in the examples of figures 8a, 9a and 8b, 9b, or with an array of pillars 911 as shown in the example of figures 8c, 9c, the array of channels 92 or pillars 911 being in contact with the gas permeable membrane 8.

The height and the spacing of the channels 92 or the pillars 911 are adjusted so as to minimize the risk of collapse of the gas permeable membrane 8 in the gas chip when the pressure in the processing chamber is greater than that in the gas exchange medium, for example by a difference of 1 bar. The channels 92 or pillars 911 supporting the gas permeable membrane 8 preferably have a height less than 100 $\mu$m, preferably less than 50 $\mu$m; an aspect ratio close to 1, preferably less than 1; and a spacing of the order of their width, for example twice their width. In the case of the use of densely arranged channels 92, the channels 92 are arranged so as to provide an efficient control of the gas concentration in the gas exchange medium present in the gas chip, through diffusion and convection. As shown in the figures, low hydraulic resistance channels may be added to the gas chip along the longitudinal axis of the segment 21 of the processing chamber to allow renewal of the gas exchange medium with a lower pressure.

When the above cross sections are chosen for the input seeding binary tree 3, the same cross sections may be chosen for the output seeding binary tree 4. In addition, when the above cross sections are chosen for the input seeding binary tree 3, the distance p between neighboring seeding channels 33 perfusing a segment 21 of the processing chamber 2 at their junction with the processing chamber may be chosen to be 1 mm, the height H of the processing chamber 2 may be chosen to be 100 $\mu$m, the width W of the processing chamber 2 may be chosen to be 5 mm.

*Continuous circulation*

As a variant of all embodiments disclosed above, a circulation pump may be used to continuously flow culture medium through the seeding flow mode. Said pump is fluidically connected to input seeding tree root 30 and output seeding tree root 40. With a continuous flow with low flow rate, medium is renewed without displacement of cells.

Such a circulation pump may be integrated to the microfluidic device, for example using miniature peristaltic pump integrated to microfluidic devices.

## METHOD

A method for processing cells using any one of the above embodiments of the microfluidic devices 1 comprises steps as described below.

*Optional step - Coating of the processing chamber*

The optional step of coating of the microfluidic device 1 is configured to result in an homogeneous coating of one or several coating reactants within the processing chamber 2.

Using the microfluidic device 1 as described above, a first coating solution is flown either by seeding flow or by harvest flow. Activating blocking means, when available, when using the harvest flows limits the coating of seeding and connecting channels. The coating solution is incubated within the microfluidic device for a variable time period, typically around 30 minutes. The device 1 is preferably kept at constant coating temperature during the procedure, said temperature being selected according to selected coating. Depending on the type of coating, the device may, prior to coating solution incubation, be activated, for example by an oxygen plasma. To achieve a homogeneous coating, it is preferable to keep the coating solution flowing during incubation, for example by back and forth flow. Particles, such as beads, sufficiently small not too clog channels but otherwise as large as possible, may be added to the coating solution, providing they can be efficiently eliminated after the coating, in order to further increase coating homogeneity by mixing flow due to particle rotation induced by the shear within the flow.

Coating may comprise the repetition of the coating step described above with several coating solutions to create a multilayered coating.

*Seeding the processing chamber*

The seeding step by means of the microfluidic device 1 is configured to result in a homogeneous distribution of cells within the processing chamber 2. It is noted that seeding should be performed relatively quickly, in particular in the case of adherent cells, in order to avoid an impact of pre-seeding conditions on the cells, such as anoikis, undesired adhesion of cells upstream the device, and/or aggregation.

Using the microfluidic device 1 as described above, the cells are seeded by seeding flow pulses, also called seeding pulses, representing a volume which is usually of the order of the volume of the processing chamber 2, usually between R = 40% and R = 200% of the volume of the processing chamber. Then, if the cells to be seeded are suspended in a volume representing x times the volume of the processing chamber 2, it takes in the order of x/R seeding flow pulses to seed them. Additional seeding pulses called secondary seeding pulses are also performed with "cell free" medium input in order to seed the cells remaining upstream, due notably to sedimentation and dispersion.

For each successive pair of seeding pulses, the pulses are separated by a resting time, or seeding pause, allowing for the most recently seeded cells to sediment in the processing chamber 2. To speed up sedimentation, and thus the seeding process, it is advantageous to seed the cells in a medium of low to moderate viscosity, for example a medium having a kinematic viscosity of not more than five times that of water at 37°C, and negligible elasticity, for example crosslinked hydrogel is not preferred as a seeding medium. For example, for a processing chamber comprising height serially arranged segments of 100 $\mu$m height, 5 mm width and 80 mm length each: the volume of the processing chamber is 320$\mu$L, the seeding flow rate can be chosen between 0.125 and 100$\mu$L/s depending notably on cell sedimentation speed. A typical value of 3.75$\mu$L/s is used as a first choice before further optimization, a seeding pause of 5s to 1200s can be used depending on the cell sedimentation speed. A typical value of between 100s and 300s is used as a first choice before further optimization. A seeding pulse of 190$\mu$L (R_dir=60%) can be chosen as a first value, assuming a widespread position distribution of the seeded cells during the seeding pulse. This value is adjusted according to the actual position distribution of the seeded cells during the seeding pulse. In such a case, one and preferably two to ten secondary seeding pulses are recommended to ensure a very low rate of cells lost upstream. The number of secondary seeding pulses should be adjusted to the total volume upstream the processing chamber 2 in the seeding flow, including potential tubing and other accessories used to perfuse the microfluidic device 1. Alternatively, a seeding pulse of 480$\mu$L (R_dir=150%) can be chosen followed by a re-verted flow of 224$\mu$L (R_rev=70%), yielding an effective ratio R=80%, as a first choice before further optimization.

To avoid a long seeding time, it is preferable that the cells to be seeded be suspended in a medium volume where x/R is lower than 20. A too low value such as x/R = 1 is not preferable either as the dispersion of the input cell suspension may be strongly affecting the homogeneity of the seeding along the width of the processing chamber. A value of x/R between 3 and 10 is recommended. With such parameters and a seeding pause of 300s, the initial seeding pulses are performed within approximately 30 minutes, which is an acceptable duration. Few cells travelling upstream more slowly for various reasons, including sedimentation and dispersion, may experience a longer duration of the seeding process which is found to not jeopardize the advantages of the invention. It is noted that this method provides a concentration of the cell suspension. This is a further advantage of the invention, since cell suspension concentration is frequently necessary in bioproduction. By integrating the concentration function within a bioprocessing device, the need for cell concentration dedicated modules is reduced, which simplifies the implementation of bioproduction protocols.

*Optional further seeding of the processing chamber*

When it is desired to perform two seeding steps, for example to seed successively two different cell populations in the same processing chamber, a second seeding step as described above can be performed after the first one. When it is not necessary to have a delay between the two seedings it is possible to use the second seeding initial pulses as secondary seeding pulses of the first seeding. By doing this, the invention advantageously allows for simultaneously mixing and concentrating two different cell populations.

Three or more seeding steps of the processing chamber can be performed sequentially and similarly to the second seeding described above. Then, again, the next seeding initial pulses can be used as secondary seeding pulses of the previous seeding.

*Optional step - Medium renewal*

Many ordinary operations of bioproduction involve partial or extensive replacement of the medium without harvesting the cells, they are called medium renewal steps hereafter.

Medium renewal steps are for example ordinarily used for cell amplification, differentiation, viral transduction (or other genetic editing method), labelling, tagging, staining, filtration, thawing, freezing (etc.) protocols.

In bioproduction, medium renewal must generally be performed accurately in terms of volumes, to result in a homogeneous medium within the processing chamber, avoid downstream loss of cells, avoid exposure to mechanical stress or other perturbations.

*Optional step - Medium renewal through the seeding channels and connection channels*

To do so with the microfluidic device 1 according to the invention, the seeding flow mode is used to distribute the flow serially in all segments of the processing chamber 2 with a minimal stress on cells as well as a minimal risk of losing cells. For partial replacement of the medium, the added volume is injected in the processing chamber 2, and then homogenized with back and forth flow or with loop flow or with a combination of both.

During such medium renewal operations, the flow rate is chosen so as to avoid excessive pressure (too high flow rate) or too slow operation (too low flow rate). The flow rate is also chosen depending on the cells so as to result in a low risk of undesired cell loss. The allowable flow rate for these medium renewal operations depends on the potential coating of the processing chamber 2 as well as on the type of the cells. It may additionally be preferable to choose a high flow rate within the range where cell loss is negligible to allow faster operation and more efficient disposal of certain compounds such as non-cellular vesicles, cell fragments, dying or dead cells.

In certain embodiments, the flow loop can be made in part or totality of gas permeable (or semi-permeable or selectively permeable) material to provide gas exchange, in particular exchange of $CO_2$ and $O_2$. This type of setup is of particular interest when the gas permeability of the processing chamber 2 is low, in order to avoid a too frequent replacement of the culture medium.

In certain embodiments, it can be found advantageous to replace a small fraction of the medium frequently rather than a large fraction less frequently to reduce the fluctuation of parameters of the medium such as nutrient concentrations. Indeed, smaller and more frequent changes result in less abrupt changes of the processing parameters which can be found to result in more robust and reproducible processes.

In certain embodiments it can be found advantageous to increase the concentration of one or several species of the medium by the replacement of the medium with a smaller amount of concentrated solution rather than a larger amount of less concentrated solution. Indeed, replacing a smaller amount of the volume can avoid unnecessary elimination of medium components the concentration of which decays more slowly than those who need to be added more frequently. Additionally, this type of operation results in less dilution of the compounds secreted by cells which can be found to have important roles to maintain or evolve cell phenotype, e.g. stem cell maintenance and differentiation.

In certain embodiments it can be found advantageous to continuously flow culture medium by use of a circulation pump.

*Optional step - Medium renewal through harvest channels*

In certain and less frequent embodiments, especially when cells are found to have a relatively high adherence to the processing chamber, the medium renewal in harvest flow mode can be found advantageous. This is notably the case when:

- a higher shear rate is desired to eliminate bodies such as dying cells, dead cells, non-cellular vesicles, cell fragments, platelets, red blood cells,
- a high shear rate is desired to trigger mechanotransduction,

In certain embodiments, these operations are performed in linear flow only, in other in loop flow only, in yet others in a combination of linear flow and loop flow.

*Optional step - Medium renewal through both types of channels*

When it is desired to completely wash the device to eliminate a compound such as a chemical, an enzyme, a virus, medium renewal using seeding and/or harvest channels may be performed simultaneously or sequentially and eventually repeatedly.

These operations contain at least some linear flow but they sometimes also comprise some loop flow.

*Optional step - Gas concentration maintenance using diffusive exchange across a gas permeable membrane closing the processing chamber*

The gas concentration in the processing chamber is advantageously obtained by diffusive exchange across a gas permeable membrane, thus allowing a reduction of culture medium consumption to compensate for oxygen consumption in the case of living cells, for example. To provide such a diffusive exchange, a gas exchange medium such a gas mix of desired composition, or a liquid with adjusted dissolved gas concentration, is disposed on the outer side of the gas permeable membrane opposite from the processing chamber. To provide stable processing conditions, or to vary processing conditions in terms of gas concentration in the processing chamber, the gas exchange medium with adjusted gas concentration is renewed on the side of the gas exchange medium opposite from the processing chamber at a rate adapted to the desired speed of variation of the gas concentration in the processing chamber or adapted to compensate for gas concentration variations in the processing chamber that may be due, for example, to metabolic activity.

In embodiments comprising a gas chip, the gas exchange medium is flown through the gas chip from its input to its output using conventional means such as pumps, gas sources or exchangers. The gas chip makes it possible to have a good control of gas concentration renewal in the processing chamber as well as a reduced required volume of gas exchange medium. This is particularly interesting when gas to be diffused into the processing chamber come from a source such as a high-pressure vessel comprising high molecular purity gas.

*Harvesting the processing chamber*

Harvesting can comprise the following steps, the order indicated below only a non-limiting example. The following steps can be performed or not, any number of times, in any order and in any sort of implementation:

-   Washing of the processing chamber 2 or of the whole microfluidic device 1 according to one of the medium renewal methods,
-   Medium renewal by injecting a cell detachment reactant into the processing chamber 2, such as an enzyme. This step can be performed according to any medium renewal method, but preferably with a method using only seeding channels 33, 43,
-   Additional physical treatment such as temperature, vibrations, electric field, magnetic field, illumination with visible or invisible light such as ultraviolet light or combinations of the latter to favor cell detachment,
-   Washing of the processing chamber 2. This step can be performed according to any medium renewal method but much preferably with a method using only seeding channels 33, 43,
-   Recollection of the cells, using only harvest channels 53, 63, while the seeding channels 33, 43 are blocked as stiffly as possible at their extremities, a flow of harvest solution such as medium or buffer being applied to the microfluidic device 1 and the cells being harvested in the output. The representative shear rate of this operation is chosen to be the highest permissible considering the constraints of mechanical integrity of the microfluidic device 1 and its accessories, with respect to the resulting pressure and the constraints of cell mechanical sensitivity. Typical shear rates allowing efficient recollection of cells with a low residual adherence to the walls of the processing chamber are in the range from 100 to 10 000s$^{-1}$; however, in specific cases, such as cells very sensitive to shear or, oppositely, cells with high residual adherence to the walls of the processing chamber, values out of this range may be used,
-   Imaging of the microfluidic device 1, and in particular of the processing chamber 2, can optionally be performed in order to evaluate the efficacy of the harvest, in particular when a lower shear rate than usual is used.

The volume flushed through the microfluidic device 1 during the recollection of the cells is at least equal to the sum of the segments 21 of the processing chamber 2, intermediary and downstream flow path volume to the recollection volume. Preferably, a volume of twice this minimal amount or greater is used. As a first value to be used before further optimization, a volume of three times the minimal volume mentioned above is recommended.

In certain embodiments, the flow rate used during the recollection of the cells is increased continuously or discontinuously. However, it is not recommended unless specific situation suggests that it would be useful. An element that may suggest the relevance of an increasing flow rate during the recollection of the cells may for example be the very high cell density within the processing chamber 2. In this case, the cell density can increase the flow rate resistance, and thus an increasing flow rate during the recollection of the cells can avoid an excessive pressure by progressively reducing the number of cells in the device. In such cases, the upstream pressure can advantageously be monitored to adjust the flow rate and avoid excessive pressure and risk of leaks.

*Optional step - Cell sorting*

Because cells may have different types of adherence in the processing chamber 2, to the coated or uncoated processing chamber or to the other cells, and because they may also have different shape and size, it is possible to exploit these differences within the microfluidic device 1 to selectively harvest certain cells and/or selectively retain certain cells in order to achieve cell sorting.

In some embodiments, the processing chamber 2 is previously coated with one or several compounds (binding anchors) such as an antibody, a nanobody, an ECM molecule, fragments of ECM molecules, peptides that are specifically binding to molecules (binding targets) which are more frequent at the surface of the cells one of the two groups of cells to be separated from each other than at the surface of the cells of the second group.

The processing chamber may be coated with a pattern comprising anti-adhesive areas and binding sites for binding anchors to increase the specificity of particle adhesion. For example, the processing chamber bottom surface (and eventually other surfaces) may be coated with PLL-PEG-biotin or silane-PEG-biotin, and a streptavidin linkage may be used to bind binding anchors such as biotinylated antibody or antibody fragments. In such cases the PEG-biotin coating may be performed priori to packaging of the device and the binding anchors may be added by the user depending on the type of sort they desire to perform. In such cases, the anti-adhesive areas limit the non-specific adhesion of particles in the chamber and thus increases the particle sorting selectivity.

In some embodiments, the cells are seeded in the processing chamber 2 according to one of the methods described above. In some embodiments, small and intense back and forth pulses are applied to the processing chamber 2 after the seeding step to reduce the frequency of superposed cells. Such pulses should represent a volume inferior to 25% of the processing chamber volume and be at least as intense as seeding flow pulses.

In some embodiments, a first recollection is performed by applying a flow through the harvest channels 53, 63 while the seeding channels 33, 43 and connection channels 73 are blocked at their extremities. In such embodiments, cells having a lower adherence to the processing chamber surface, coating or to other cells that are already adhering quite strongly are preferentially recollected, resulting in enrichment in such cells in the output of the flow and a depletion of those cells within the processing chamber 2. In some such embodiments, the flow rate is increased continuously or discontinuously up to values slightly inferior to those compromising the viability of the cells of interest or to those compromising the apparatus mechanical integrity. In some such embodiments, the volume obtained from the processing chamber 2 during this flow is separated according to ranges of applied flow rate. In some such embodiments, the number of cells recollected per unit time is monitored during this operation, for example using an imaging sensor positioned at the output of the harvest channel 63. In some such embodiments, the measure of the number of cells recollected per unit time is used to tune the flow rate. In some such embodiments, the flow rate is automatically increased until the maximum value at a specific rate while the measure of the number of cells recollected per unit time is inferior to a previously defined threshold value.

In some embodiments, a harvest (second recollection) according to one of the harvest methods described above is performed after the first sorting flow is applied, to recollect some, potentially all, of the cells remaining in the processing chamber 2.

In some embodiments, one of the sorted fractions collected during the first or the second recollection is seeded again in the processing chamber 2, or in the processing chamber of a similar microfluidic device, to undergo an additional sorting. Any number of such sorting repetitions can be performed to obtain the desired composition and purity of the cell population. Optionally, different binding sites may be used to sort cells on multiple features.

As can be seen from the previous examples, a microfluidic device according to the invention provides a novel geometry where a processing chamber of high capacity is connected to two sets of channels corresponding to two distinct modes of flow, i.e. a seeding flow mode and a harvest flow mode. The seeding flow mode makes it possible to obtain a homogeneous and efficient seeding of the processing chamber, with low risk of upstream and downstream particle deposition, and makes the microfluidic device capable of concentrating and washing a particle suspension, while the harvest flow mode provides a quick, efficacious and efficient harvest. By integrating these functionalities into one microfluidic device, the invention is particularly adapted to implement automated and/or miniaturized bioproduction, which has many technical advantages such as gain in particle and reactant efficiency due to the reduction of the number of transfer steps. This unique configuration has the additional advantage of being feasible with only one channel layer, without filter membranes, thus drastically reducing manufacturing complexity and cost.

The invention is not limited to the examples described and shown, but the scope of protection is limited by the claims.

In particular, in the illustrative embodiments described above, the microfluidic device comprises a single input seeding tree and a single output seeding tree for several segments of the processing chamber, connected with connection channels.

Furthermore, in the examples shown in the figures, the microfluidic device does not comprise chamfers at the junctions of the input seeding channels, output seeding channels and connection channels with the processing chamber. The presence of such chamfers is however advantageous and within the scope of the invention. Alternative progressive increase of channel cross-section in the vicinity and toward the processing chamber, such as a fillet, can also be found advantageous to improve the seeding flow.

Additionally, in the embodiments described above, each segment of the processing chamber has a rectangular geometry and seeding trees are symmetric binary trees of seeding channels. However, in other embodiments of the invention, the processing chamber and the trees of seeding channels may have different shapes and arrangements. **In** some embodiments of the invention, several microfluidic devices according to the invention may also be stacked, the devices of the stack then being possibly connected by hydraulic manifolds.

According to another variant, the processing chamber may contain pillars joining its top and bottom surfaces to reduce the risk of collapse related to the flatness of the processing chamber. Such additional pillars may be particularly advantageous in embodiments where the width of the processing chamber is much greater than its height.

**Claims**

1. Microfluidic device (1) for processing particles, in particular cells, comprising:

 - a processing chamber (2) including at least two elongated segments (21);
 - at least one input seeding channel (33) and one output seeding channel (43) configured to define a seeding flow in a transverse direction (Y) to the longitudinal direction (X) of all segments (21) of the processing chamber (2); and
 - at least one input harvest channel (53) and one output harvest channel (63) configured to define a harvest flow in the longitudinal direction (X) of all segments (21) of the processing chamber (2),

wherein the microfluidic device (1) comprises:

- for a first segment S1 (21) of the processing chamber (2), a plurality of input seeding channels (33) whose junctions (31) with S1 are distributed along S1, the plurality of input seeding channels (33) being defined by a single input seeding tree (3);
- for a second segment S2 (21) of the processing chamber (2) different from S1, a plurality of output seeding channels (43) whose junctions (41) with S2 are distributed along S2, the plurality of output seeding channels (43) being defined by a single output seeding tree (4); and
- one connection channel (73) or a plurality of connection channels (73), each connection channel having junctions (71) with two distinct segments (21) of the processing chamber, the connection channel (73) or plurality of connection channels (73) being configured to allow the seeding flow through all the segments (21) serially.

2. Microfluidic device according to claim **1,** comprising blocking means configured to block selectively the input and output harvest channels (53, 63) when a seeding flow is applied to the processing chamber (2) through the input seeding channels, connection channels and output seeding channels (33, 43, 73).

3. Microfluidic device according to claim **1** or claim **2,** comprising blocking means configured to block selectively the input seeding channels and output seeding channels (33, 43, 73) when a harvest flow is applied to the processing chamber (2) through the input and output harvest channels (53, 63).

4. Microfluidic device according to any one of the preceding claims, comprising blocking means configured to block selectively the connection channels (73) when a harvest flow is applied to the processing chamber (2) through the input and output harvest channels (53, 63).

5. Microfluidic device according to any one of the preceding claims, wherein the surface of the processing chamber (2), perpendicular to the height direction (Z), is greater than 4 cm$^2$, preferably greater than 10 cm$^2$, more preferably greater than 16 cm$^2$, more preferably greater than 20 cm$^2$, more preferably greater than 30 cm$^2$.

6. Microfluidic device according to any one of the preceding claims, wherein, the pitch (p) between adjacent junctions (31) of the input seeding channels (33) with the first segment (21) of the processing chamber (2) and the pitch (p) between adjacent junctions (41) of the output seeding channels (43) with the second segment (21) of the processing chamber (2) is the same.

7. Microfluidic device according to any one of the preceding claims, wherein, the pitch (p) between adjacent junctions (71) of the connecting channels (73) with the segments (21) and the pitch (p) between adjacent junctions (31) of the input seeding channels (33) with the first segment (21) of the processing chamber (2) is the same.

8. Microfluidic device according to any one of the preceding claims, wherein connecting channels (73) have all the same length.

9. Microfluidic device according to any one of the preceding claims, wherein, in each seeding tree (3, 4), the cross section of the seeding channels (33, 43) decreases with increasing channel path distance from the tree root (30, 40).

10. Microfluidic device according to any one of the preceding claims, wherein the ratio of the cross section ($S_{21}$) perpendicular to the transverse direction (Y) of the first segment (21) of the processing chamber (2) to the sum of the average cross sections ($S_{31}$) of the input seeding channels (33) perfusing the first segment (21) is higher than 5, preferably higher than 10, more preferably higher than 15.

11. Microfluidic device according to any one of the preceding claims, wherein the total volume of the seeding trees (3, 4) is less than the total volume of the processing chamber (2), preferably less than 33% of the total volume of the processing chamber (2), more preferably less than 10% of the total volume of the processing chamber (2).

12. Microfluidic device according to any one of the preceding claims, comprising fluidic connectors (24) between segments (21) of the processing chamber (2), said fluidic connectors (24) being configured to allow the harvest flow through all the segments (21) of the processing chamber (2) serially.

13. Method for processing particles, in particular cells, using a microfluidic device (1) according to any one of the preceding claims, the method comprising:

- a step of seeding serially the segments (21) of the processing chamber (2) with particles by applying a seeding flow to the processing chamber (2) through the input seeding channel (33), connection channels (73) and output seeding channels (43), while the input and output harvest channels (53, 63) are blocked,

- a step of collecting particles from the processing chamber (2) by applying a harvest flow to the processing chamber (2) through the input and output harvest channels (53, 63), while the input seeding channel (33), connection channels (73) and output seeding channels (43) are blocked.

14. Method according to claim **13,** wherein the step of seeding serially the segments (21) of the processing chamber (2) is carried out by applying successive pulses of seeding flow separated by a resting time.

15. Method according to claim **13** or claim **14,** wherein the step of collecting particles from the processing chamber (2) is carried out by applying successively different flow rates of the harvest flow.

**Patentansprüche**

1. Mikrofluidische Vorrichtung (1) zum Bearbeiten von Partikeln, insbesondere Zellen, umfassend:

> - eine Bearbeitungskammer (2), die mindestens zwei längliche Segmente (21) umfasst;
> - mindestens einen Eingangssaatkanal (33) und einen Ausgangssaatkanal (43), die so eingerichtet sind, dass sie einen Saatfluss in einer Querrichtung (Y) zur Längsrichtung (X) aller Segmente (21) der Bearbeitungskammer (2) definieren; und
> - mindestens einen Eingangserntekanal (53) und einen Ausgangserntekanal (63), die so eingerichtet sind, dass sie einen Erntefluss in der Längsrichtung (X) aller Segmente (21) der Bearbeitungskammer (2) definieren,

> wobei die mikrofluidische Vorrichtung (1) Folgendes umfasst:

> - für ein erstes Segment S1 (21) der Bearbeitungskammer (2) eine Vielzahl von Eingangssaatkanälen (33), deren Verbindungen (31) mit S1 entlang S1 verteilt sind, wobei die Vielzahl von Eingangssaatkanälen (33) durch einen einzigen Eingangssaatbaum (3) definiert ist;
> - für ein zweites Segment S2 (21) der Bearbeitungskammer (2), das sich von S1 unterscheidet, eine Vielzahl von Ausgangssaatkanälen (43), deren Verbindungen (41) mit S2 entlang S2 verteilt sind, wobei die Vielzahl von Ausgangssaatkanälen (43) durch einen einzigen Ausgangssaatbaum (4) definiert ist; und
> - einen Verbindungskanal (73) oder eine Vielzahl von Verbindungskanälen (73), wobei jeder Verbindungskanal Verbindungen (71) mit zwei getrennten Segmenten (21) der Bearbeitungskammer aufweist, wobei der Verbindungskanal (73) oder die Vielzahl von Verbindungskanälen (73) so eingerichtet sind, dass sie den Saatfluss seriell durch alle Segmente (21) zulassen.

2. Mikrofluidische Vorrichtung nach Anspruch **1,** umfassend Blockiermittel, die so eingerichtet sind, dass sie die Eingangs- und Ausgangserntekanäle (53, 63) selektiv blockieren, wenn ein Saatfluss der Bearbeitungskammer (2) durch die Eingangssaatkanäle, Verbindungskanäle und Ausgangssaatkanäle (33, 43, 73) zugeführt wird.

3. Mikrofluidische Vorrichtung nach Anspruch **1** oder Anspruch **2,** umfassend Blockiermittel, die so eingerichtet sind, dass sie die Eingangssaatkanäle und die Ausgangssaatkanäle (33, 43, 73) selektiv blockieren, wenn ein Erntefluss der Bearbeitungskammer (2) durch die Eingangs- und Ausgangserntekanäle (53, 63) zugeführt wird.

4. Mikrofluidische Vorrichtung nach einem der vorhergehenden Ansprüche, umfassend Blockiermittel, die so eingerichtet sind, dass sie die Verbindungskanäle (73) selektiv blockieren, wenn ein Erntefluss der Bearbeitungskammer (2) durch die Eingangs- und Ausgangserntekanäle (53, 63) zugeführt wird.

5. Mikrofluidische Vorrichtung nach einem der vorhergehenden Ansprüche, wobei die Oberfläche der Bearbeitungskammer (2), senkrecht zur Höhenrichtung (Z), größer als 4 cm$^2$, bevorzugt größer als 10 cm$^2$, bevorzugter größer als 16 cm$^2$, bevorzugter größer als 20 cm$^2$, bevorzugter größer als 30 cm$^2$, ist.

6. Mikrofluidische Vorrichtung nach einem der vorhergehenden Ansprüche, wobei der Abstand (p) zwischen benachbarten Verbindungen (31) der Eingangssaatkanäle (33) mit dem ersten Segment (21) der Bearbeitungskammer (2) und der Abstand (p) zwischen benachbarten Verbindungen (41) der Ausgangssaatkanäle (43) mit dem zweiten Segment (21) der Bearbeitungskammer (2) gleich ist.

7. Mikrofluidische Vorrichtung nach einem der vorhergehenden Ansprüche, wobei der Abstand (p) zwischen benachbarten Verbindungen (71) der Verbindungskanäle (73) mit den Segmenten (21) und der Abstand (p) zwischen benachbarten Verbindungen (31) der Eingangssaatkanäle (33) mit dem ersten Segment (21) der Bearbeitungskammer (2) gleich ist.

8. Mikrofluidische Vorrichtung nach einem der vorhergehenden Ansprüche, wobei die Verbindungskanäle (73) alle dieselbe Länge aufweisen.

**9.** Mikrofluidische Vorrichtung nach einem der vorhergehenden Ansprüche, wobei in jedem Saatbaum (3, 4) der Querschnitt der Saatkanäle (33, 43) mit zunehmender Kanalpfadentfernung von der Baumwurzel (30, 40) abnimmt.

**10.** Mikrofluidische Vorrichtung nach einem der vorhergehenden Ansprüche, wobei das Verhältnis des Querschnitts ($S_{21}$) senkrecht zur Querrichtung (Y) des ersten Segments (21) der Bearbeitungskammer (2) zur Summe der durchschnittlichen Querschnitte ($S_{31}$) der Eingangssaatkanäle (33), die das erste Segment (21) perfundieren, höher als 5, vorzugsweise höher als 10, noch bevorzugter höher als 15, ist.

**11.** Mikrofluidische Vorrichtung nach einem der vorhergehenden Ansprüche, wobei das Gesamtvolumen der Saatbäume (3, 4) weniger als das Gesamtvolumen der Bearbeitungskammer (2), vorzugsweise weniger als 33 % des Gesamtvolumens der Bearbeitungskammer (2), noch bevorzugter weniger als 10 % des Gesamtvolumens der Bearbeitungskammer (2), beträgt.

**12.** Mikrofluidische Vorrichtung nach einem der vorhergehenden Ansprüche, umfassend fluidische Anschlüsse (24) zwischen Segmenten (21) der Bearbeitungskammer (2), wobei die fluidischen Anschlüsse (24) so eingerichtet sind, dass sie den Erntefluss seriell durch alle Segmente (21) der Bearbeitungskammer (2) zulassen.

**13.** Verfahren zum Bearbeiten von Partikeln, insbesondere Zellen, unter Verwendung einer mikrofluidischen Vorrichtung (1) nach einem der vorhergehenden Ansprüche, wobei das Verfahren Folgendes umfasst:

- einen Schritt des seriellen Besäens der Segmente (21) der Bearbeitungskammer (2) mit Partikeln durch Anlegen eines Saatflusses an die Bearbeitungskammer (2) durch den Eingangssaatkanal (33), die Verbindungskanäle (73) und die Ausgangssaatkanäle (43), während die Eingangs- und Ausgangserntekanäle (53, 63) blockiert sind,
- einen Schritt des Sammelns von Partikeln aus der Bearbeitungskammer (2) durch Anlegen eines Ernteflusses an die Bearbeitungskammer (2) durch die Eingangs- und Ausgangserntekanäle (53, 63), während der Eingangssaatkanal (33), die Verbindungskanäle (73) und die Ausgangssaatkanäle (43) blockiert sind.

**14.** Verfahren nach Anspruch **13,** wobei der Schritt des seriellen Besäens der Segmente (21) der Bearbeitungskammer (2) durch Anlegen aufeinanderfolgender Impulse des Saatflusses, die durch eine Ruhezeit getrennt sind, durchgeführt wird.

**15.** Verfahren nach Anspruch **13** oder **14,** wobei der Schritt des Sammelns von Partikeln aus der Bearbeitungskammer (2) durch sukzessives Anwenden unterschiedlicher Durchflussraten des Ernteflusses durchgeführt wird.

**Revendications**

**1.** Dispositif microfluidique (1) pour le traitement de particules, notamment de cellules, comprenant :

- une chambre de traitement (2) comprenant au moins deux segments allongés (21);
- au moins un canal d'ensemencement d'entrée (33) et un canal d'ensemencement de sortie (43) configurés pour définir un flux d'ensemencement dans une direction transversale (Y) par rapport à la direction longitudinale (X) de tous les segments (21) de la chambre de traitement (2) ; et
- au moins un canal de récolte d'entrée (53) et un canal de récolte de sortie (63) configurés pour définir un flux de récolte dans la direction longitudinale (X) de tous les segments (21) de la chambre de traitement (2),

dans lequel le dispositif microfluidique (1) comprend:

- pour un premier segment S1 (21) de la chambre de traitement (2), une pluralité de canaux d'ensemencement d'entrée (33) dont les jonctions (31) avec S1 sont réparties le long de S1, la pluralité de canaux d'ensemencement d'entrée (33) étant définie par un seul arbre d'ensemencement d'entrée (3) ;
- pour un deuxième segment S2 (21) de la chambre de traitement (2) différent de S1, une pluralité de canaux d'ensemencement de sortie (43) dont les jonctions (41) avec S2 sont réparties le long de S2, la pluralité de canaux d'ensemencement de sortie (43) étant définie par un seul arbre d'ensemencement de sortie (4) ; et
- un canal de connexion (73) ou une pluralité de canaux de connexion (73), chaque canal de connexion ayant des jonctions (71) avec deux segments distincts (21) de la chambre de traitement, le canal de connexion (73) ou la pluralité de canaux de connexion (73) étant configurés pour permettre le flux d'ensemencement à travers tous les segments (21) en série.

**2.** Dispositif microfluidique selon la revendication 1, comprenant des moyens de blocage configurés pour bloquer sélectivement les canaux de récolte d'entrée et de sortie (53, 63) lorsqu'un flux d'ensemencement est appliqué à la chambre de traitement (2) à travers les canaux d'ensemencement d'entrée, les canaux de connexion et les canaux d'ensemencement de sortie (33, 43, 73).

3. Dispositif microfluidique selon la revendication 1 ou la revendication 2, comprenant des moyens de blocage configurés pour bloquer sélectivement les canaux d'ensemencement d'entrée et les canaux d'ensemencement de sortie (33, 43, 73) lorsqu'un flux de récolte est appliqué à la chambre de traitement (2) par l'intermédiaire des canaux de récolte d'entrée et de sortie (53, 63).

4. Dispositif microfluidique selon l'une quelconque des revendications précédentes, comprenant des moyens de blocage configurés pour bloquer sélectivement les canaux de connexion (73) lorsqu'un flux de récolte est appliqué à la chambre de traitement (2) par l'intermédiaire des canaux de récolte d'entrée et de sortie (53, 63).

5. Dispositif microfluidique selon l'une quelconque des revendications précédentes, dans lequel la surface de la chambre de traitement (2), perpendiculaire à la direction de la hauteur (Z), est supérieure à 4 cm$^2$, de préférence supérieure à 10 cm$^2$, plus particulièrement supérieure à 16 cm$^2$, plus particulièrement supérieure à 20 cm$^2$, plus particulièrement supérieure à 30 cm$^2$.

6. Dispositif microfluidique selon l'une quelconque des revendications précédentes, dans lequel le pas (p) entre les jonctions adjacentes (31) des canaux d'ensemencement d'entrée (33) avec le premier segment (21) de la chambre de traitement (2) et le pas (p) entre les jonctions adjacentes (41) des canaux d'ensemencement de sortie (43) avec le deuxième segment (21) de la chambre de traitement (2) est le même.

7. Dispositif microfluidique selon l'une quelconque des revendications précédentes, dans lequel le pas (p) entre les jonctions adjacentes (71) des canaux de connexion (73) avec les segments (21) et le pas (p) entre les jonctions adjacentes (31) des canaux d'ensemencement d'entrée (33) avec le premier segment (21) de la chambre de traitement (2) est le même.

8. Dispositif microfluidique selon l'une quelconque des revendications précédentes, dans lequel les canaux de connexion (73) ont tous la même longueur.

9. Dispositif microfluidique selon l'une quelconque des revendications précédentes, dans lequel, dans chaque arbre de semis (3, 4), la section transversale des canaux de semis (33, 43) diminue avec l'augmentation de la distance du chemin du canal à partir de la racine de l'arbre (30, 40)

10. Dispositif microfluidique selon l'une quelconque des revendications précédentes, dans lequel le rapport de la section transversale (S21) perpendiculaire à la direction transversale (Y) de la première le segment (21) de la chambre de traitement (2) à la somme des sections transversales moyennes (S31) des canaux d'ensemencement d'entrée (33) perfusant le premier segment (21) est supérieur à 5, de préférence supérieur à 10, plus particulièrement supérieur à 15.

11. Dispositif microfluidique selon l'une quelconque des revendications précédentes, dans lequel le volume total des arbres de semis (3, 4) est inférieur au volume total de la chambre de traitement (2), de préférence inférieur à 33 % du volume total de la chambre de traitement (2), plus particulièrement inférieur à 10 % du volume total de la chambre de traitement (2).

12. Dispositif microfluidique selon l'une quelconque des revendications précédentes, comprenant des connecteurs fluidiques (24) entre les segments (21) de la chambre de traitement (2), lesdits connecteurs fluidiques (24) étant configurés pour permettre au flux de récolte de traverser en série tous les segments (21) de la chambre de traitement (2).

13. Procédé de traitement de particules, notamment de cellules, utilisant un dispositif microfluidique (1) selon l'une quelconque des revendications précédentes, le procédé comprenant:

   - une étape d'ensemencement en série des segments (21) de la chambre de traitement (2) avec des particules en appliquant un flux d'ensemencement à la chambre de traitement (2) à travers le canal d'ensemencement d'entrée (33), les canaux de connexion (73) et les canaux d'ensemencement de sortie (43), tandis que les canaux de récolte d'entrée et de sortie (53, 63) sont bloqués,
   - une étape de collecte des particules de la chambre de traitement (2) en appliquant un flux de récolte à la chambre de traitement (2) à travers les canaux de récolte d'entrée et de sortie (53, 63), tandis que le canal d'ensemencement d'entrée (33), les canaux de connexion (73) et les canaux d'ensemencement de sortie (43) sont bloqués.

14. Procédé selon la revendication 13, dans lequel l'étape d'ensemencement en série des segments (21) de la chambre de traitement (2) est réalisée en appliquant des impulsions successives de flux d'ensemencement séparées par un temps de repos.

**15.** Procédé selon la revendication 13 ou la revendication 14, dans lequel l'étape de collecte des particules de la chambre de traitement (2) est réalisée en appliquant successivement différents débits du flux de récolte.

**FIG. 1**

FIG. 2

**FIG. 3**

**FIG. 4a**

**FIG. 4b**

**FIG. 5**

**FIG. 6**

**FIG. 7**

FIG. 8a          FIG. 8b          FIG. 8c

FIG. 9a          FIG. 9b          FIG. 9c

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010035292 A1